# EUROPEAN PATENT APPLICATION

(11) **EP 4 317 979 A2**
(43) Date of publication of application: **07.02.2024**
(21) Application number: 22776016.2
(22) Date of filing: 21.03.2022
(51) Int. Cl.: G01N 33/68, G01N 33/50, C12Q 1/6883, A61K 31/675, A61P 35/00, A23L 33/10

(54) **COMPOSITION FOR DIFFERENTIAL DIAGNOSIS OF MALIGNANT PERIPHERAL NERVE SHEATH TUMOR**

(30) Priority: 24.03.2021 KR 20210037912; 18.03.2022 KR 20220034295
(71) Applicant: The Asan Foundation, Seoul 05505 (KR); University Of Ulsan Foundation For Industry Cooperation, Ulsan 44610 (KR); Humanscape Inc., Gangnam-gu Seoul 06163 (KR)
(72) Inventor: KIM, Chong Jai, Seoul 05392 (KR); KIM, Kyung Gon, Seoul 05507 (KR); LEE, Beom Hee, Seoul 05530 (KR); KIM, Eunna, Yongin-si Gyeonggi-do 17084 (KR); CHANG, Min Hoo, Seoul 06196 (KR); JEONG, Hwangkyo, Yongin-si Gyeonggi-do 16884 (KR); DO, Hyo-Sang, Seoul 05333 (KR)
(74) Representative: Dehns
(86) International application number: PCT/KR2022/003918
(87) International publication number: WO 2022/203314

(57) **Abstract**

The present invention relates to a composition for the differential diagnosis of malignant peripheral nerve sheath tumor (MPNST), a differential diagnosis kit comprising same, a method for providing information required for differential diagnosis of MPNST using same, a method for screening materials for preventing, alleviating, and treating MPNST, and the like. Using the composition capable of detecting the protein marker of the present invention has the effect of making it possible to predict and diagnose whether a malignant tumor has occurred in a patient with neurofibromatosis type 1.

## Description

### [Technical Field]

The present invention relates to a composition for the differential diagnosis of malignant peripheral nerve sheath tumor (MPNST), a differential diagnostic kit including the same, a method of providing information required for differential diagnosis of MPNST using the same, and a method of screening materials for preventing, alleviating and treating MPNSTs.

The present invention claims priority to and the benefit of Korean Patent Application No. 10-2021-0037912, filed on March 24, 2021, and Korean Patent Application No. 10-2022-0034295, filed on March 18, 2022, the disclosures of which are incorporated herein by reference in their entirety.

### [Background Art]

Neurofibromatosis type I (hereinafter, referred to as "NF-1") was first described by Fredrich von Recklinghausen in 1882, and is one of the most common genetic disorders. NF-1 is inherited via autosomal dominant inheritance, affecting 1:3,000 people around the world. NF-1 is characterized by multiple cafe-au-lait-spots on the skin and neurofibromas in the skin. In addition, various levels of neurological, skeletal, and neoplastic manifestations may affect patients. NF-1 patients are at risk for central and peripheral nervous system tumors, including plexiform neurofibromas (PN; 27%), optic nerve gliomas (15-20%), pheochromocytomas (1%), and malignant peripheral nerve sheath tumors (MPNSTs; 8-13%). This is approximately 1,000 times higher than the general population.

Malignant peripheral nerve sheath tumor (hereinafter, referred to as "MPNST") is one of the complications that have the greatest impact on the survival rate of NF-1 patients. It is known to be caused by malignant transformation of PN, which is a benign myelin sheath tumor, and must be suspected when severe pain, a dramatic increase in the size of fibrosis, and neurological symptoms appear.

Until now, 18F-fluorodeoxyglucose positron emission computerized tomography (18F-FDG-PET) has been used to distinguish a benign tumor from MPNSTs, and when a malignancy is suspected, MPNST is diagnosed based on histological findings.

In most MPNSTs, histologically, bundles of spindle cells are formed, cells with nuclear differentiation are observed and infiltrate surrounding tissue. To diagnose MPNST, S100 protein staining is used, shows positive in 50 to 90% of MPNSTs, and in addition, the Leu-7 protein and myelin basic protein show positive in approximately 50% of MPNSTs. Vimentin is also a pathological marker used for MPNST diagnosis, but is specific for spindle cells and is limited in MPNST-specific diagnosis. Therefore, there is a need for pathological indicators that can clearly determine the progression of MPNST in NF-1.

MPNST is a disease requiring surgical resection after diagnosis. After surgery, approximately 50% of MPNSTs have a risk of recurrence, and chemotherapy and radiation therapy may be required when complete resection is not achieved by surgery. Nevertheless, the 5-year survival rate for MPNST patients remains at 20 to 50%.

In the absence of a treatment method that can target MPNST so far, it is considered that finding a biomarker that can early diagnose MPNST in blood or urine can contribute to improving the survival rate of patients.

### [Disclosure]

### [Technical Problem]

As a result of performing extensive quantitative and qualitative proteomic analyses with malignant peripheral nerve sheath tumor (MPNST) and benign neurofibroma tissue, the present inventors identified proteins specifically expressed in MPNST tissue, and thus completed the present invention.

Therefore, the present invention is directed to providing a composition for differential diagnosis of MPNST.

The present invention is also directed to providing a kit for differential diagnosis of MPNST, which includes the composition according to the present invention.

The present invention is also directed to providing a method of providing information required for the differential diagnosis of MPNST.

The present invention is also directed to providing a composition for preventing, alleviating or treating MPNST.

The present invention is also directed to providing a method of screening a material for preventing, alleviating or treating MPNST.

However, technical problems to be solved in the present invention are not limited to the above-described problems, and other problems which are not described herein will be fully understood by those of ordinary skill in the art from the following description.

### [Technical Solution]

To achieve the purpose of the present invention, the present invention provides a composition for differential diagnosis of MPNST, which includes an agent that measures the expression level(s) of one or more proteins selected from the group consisting of endosialin, SPARC-like protein 1, and neutrophil collagenase or the expression level of mRNA thereof as an active ingredient.

In one embodiment of the present invention, the composition may diagnose and distinguish MPNST from neurofibromatosis type I (NF-1).

In another embodiment of the present invention, the agent that measures an expression level of the protein may be an antibody or aptamer specific for the protein, but the present invention is not limited thereto.

In still another embodiment of the present invention, the agent that measures the expression level of the mRNA may be a primer set or probe, which specifically binds to the mRNA, but the present invention is not limited thereto.

In addition, the present invention provides a kit for differential diagnosis of MPNSTs, which includes the composition according to the present invention.

In one embodiment of the present invention, the kit may include instructions for determining a case as MPNST when the expression of one or more proteins selected from the group consisting of endosialin, SPARC-like protein 1, and neutrophil collagenase or the expression of mRNA thereof is detected, or when the expression level thereof is higher than that of a benign neurofibromatosis sample.

In addition, the present invention provides a method of providing information required for differential diagnosis of MPNST, which includes measuring the expression level(s) of one or more proteins selected from the group consisting of endosialin, SPARC-like protein 1, and neutrophil collagenase, or the expression level of mRNA thereof in a biological sample isolated from a subject.

In addition, the present invention provides a method of performing differential diagnosis of MPNSTs, which includes measuring the expression level(s) of one or more proteins selected from the group consisting of endosialin, SPARC-like protein 1, and neutrophil collagenase, or the expression level of mRNA thereof in a biological sample isolated from a subject.

In one embodiment of the present invention, the method may further include diagnosing a case as MPNST when the expression of one or more proteins selected from the group consisting of endosialin, SPARC-like protein 1, and neutrophil collagenase or the expression of mRNA thereof is detected, or when the expression level thereof is higher than that of a biological sample isolated from an NF-1 patient.

In another embodiment of the present invention, the subject may be a patient suspected of having MPNST or diagnosed with NF-1.

In still another embodiment of the present invention, the biological sample may be one or more selected from the group consisting of blood, whole blood, plasma, urine, tissue, cells, an organ, bone marrow, a microneedle aspiration specimen, a microneedle washing fluid, a core needle biopsy specimen, and saliva, but the present invention is not limited thereto.

In yet another embodiment of the present invention, the protein expression level may be measured by one or more methods selected from the group consisting of western blotting, enzyme linked immunosorbent assay (ELISA), radioimmunoassay (RIA), radioimmunodiffusion, Ouchterlony immunodiffusion, Rocket immunoelectrophoresis, tissue immunostaining, immunoprecipitation assay, complement fixation assay, fluorescence-activated cell sorting (FACS) and a protein chip, but the present invention is not limited thereto.

In yet another embodiment of the present invention, the mRNA expression level may be measured by one or more methods selected from the group consisting of PCR, RNase protection assay, northern blotting, southern blotting, in situ hybridization, and a DNA chip, but the present invention is not limited thereto.

In addition, the present invention provides a pharmaceutical composition for preventing or treating MPNST, which includes an inhibitor of the activity of one or more proteins involved in signaling pathways selected from the group consisting of the TGF-β signaling pathway, the ILK signaling pathway, the actin-cytoskeleton signaling pathway, and the RhoGDI signaling pathway, or an inhibitor of the expression of a gene encoding the protein as an active ingredient.

Further, the present invention provides a method of preventing or treating MPNSTs, which includes administering an inhibitor of the activity of one or more proteins involved in signaling pathways selected from the group consisting of the TGF-β signaling pathway, the ILK signaling pathway, the actin-cytoskeleton signaling pathway, and the RhoGDI signaling pathway, or an inhibitor of the expression of a gene encoding the protein to a subject in need thereof.

Moreover, the present invention provides a use of an inhibitor of the activity of one or more proteins involved in signaling pathways selected from the group consisting of the TGF-β signaling pathway, the ILK signaling pathway, the actin-cytoskeleton signaling pathway, and the RhoGDI signaling pathway, or an inhibitor of the expression of a gene encoding the protein for preventing or treating MPNSTs.

In addition, the present invention provides a health functional food composition for preventing or improving MPNSTs, which includes an inhibitor of the activity of one or more proteins involved in signaling pathways selected from the group consisting of the TGF-β signaling pathway, the ILK signaling pathway, the actin-cytoskeleton signaling pathway, and the RhoGDI signaling pathway, or an inhibitor of the expression of a gene encoding the protein as an active ingredient.

In one embodiment of the present invention, the signaling protein may be a kinase, but the present invention is not limited thereto.

In another embodiment of the present invention, the kinase may be a kinase encoded by one or more genes selected from the group consisting of CDK4, STK10, CDK5, MAP2K1, ALPK3, PNKP, PDK3, PHKG1, CFL1, and PACSIN3, but the present invention is not limited thereto.

In still another embodiment of the present invention, the protein activity inhibitor may be one or more selected from the group consisting of a peptide, an antibody, an aptamer, and a compound, which specifically bind to the protein, but the present invention is not limited thereto.

In yet another embodiment of the present invention, the signaling protein activity inhibitor may be one or more selected from the group consisting of selumetinib, alvocidib, purvalanol, palbociclib, ribociclib, abemaciclib, fostamatinib, binimetinib, radicicol, AZD-8330, dihydrolipoic acid, alsterpaullone, hymenialdisine, indirubin-3'-monoxime, olomoucine, SU9516, and 6-phenyl[5H]pyrrolo[2,3-B]pyrazine, but the present invention is not limited thereto.

In yet another embodiment of the present invention, the gene expression inhibitor may be one or more selected from the group consisting of miRNA, siRNA, shRNA, a ribozyme, a DNAzyme, a peptide nucleic acid (PNA), and an antisense oligonucleotide, which complementarily bind to mRNA of the gene, but the present invention is not limited thereto.

In addition, the present invention provides a method of screening a material for preventing, alleviating or treating MPNSTs, which includes: (a) bringing a candidate material into contact with isolated cells or tissue; (b) measuring the activity of one or more signaling protein(s) involved in signaling pathways selected from the group consisting of the TGF-β signaling pathway, the ILK signaling pathway, the actin-cytoskeleton signaling pathway, and the RhoGDI signaling pathway or an expression level of gene(s) encoding the protein(s) in the cells or tissue; and (c) determining the candidate as a material for preventing, alleviating or treating MPNST when the activity of the protein or the expression level of a gene encoding the protein is reduced in cells or tissue in contact with the candidate material, compared to that before contact with the candidate material.

In one embodiment of the present invention, the signaling protein may be a kinase, but the present invention is not limited thereto.

In another embodiment of the present invention, the kinase may be a kinase encoded by one or more genes selected from the group consisting of CDK4, STK10, CDK5, MAP2K1, ALPK3, PNKP, PDK3, PHKG1, CFL1, and PACSIN3, but the present invention is not limited thereto.

In still another embodiment of the present invention, the isolated cells or tissue may be nerve cells or nerve tissue, but the present invention is not limited thereto.

### [Advantageous Effects]

The present invention relates to a composition for differential diagnosis of malignant peripheral nerve sheath tumors (MPNSTs), and a composition capable of detecting a protein marker of the present invention can be used for the prediction, diagnosis, and treatment of a malignant tumor in a neurofibromatosis type I (NF-1) patient.

### [Description of Drawings]

FIGS. 1A and 1B are the time-series flowcharts of quantitative proteomic analysis using benign neurofibromatosis (NF) and malignant peripheral nerve sheath tumor (MPNST) tissue.
FIG. 2 is a diagram illustrating a proteomic data analysis method.
FIG. 3 is a graph of the distribution of protein abundance illustrating the quantitative result of proteins for NF and MPNST tissue.
FIG. 4 shows the result of qualitative proteomic analysis for two groups of NF and MPNST using Venn diagram analysis.
FIGS. 5A and 5B show the results of investigating proteins with a 10-fold increase in expression in NF and MPNST tissue, in which FIG. 5A shows the result of selecting a protein expressed in extracellular exosomes, and FIG. 5B shows the result of selecting a protein degrading the extracellular matrix.

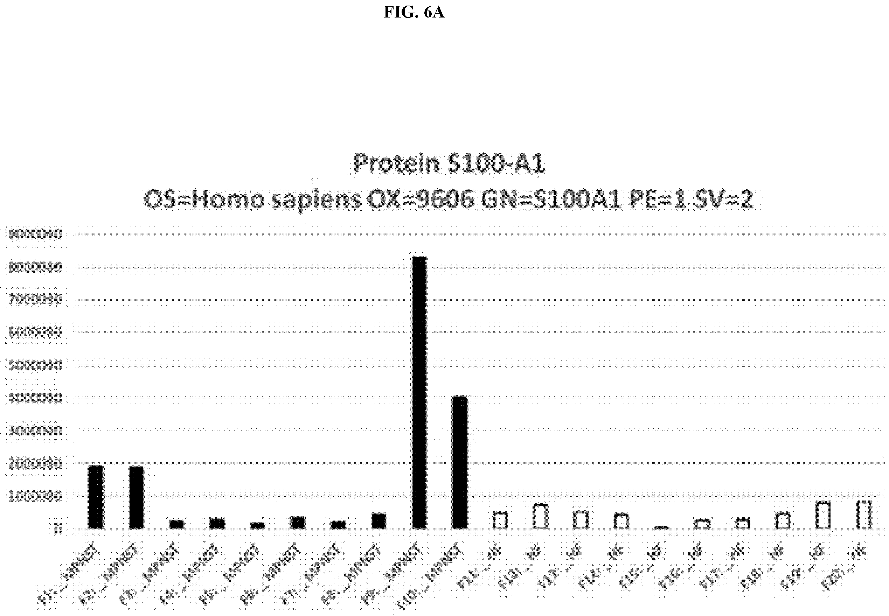

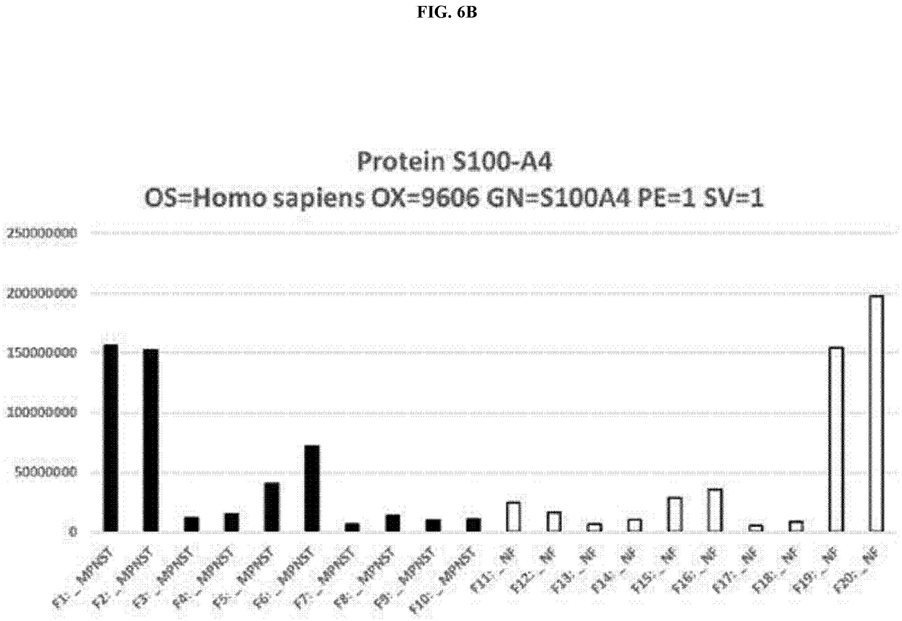

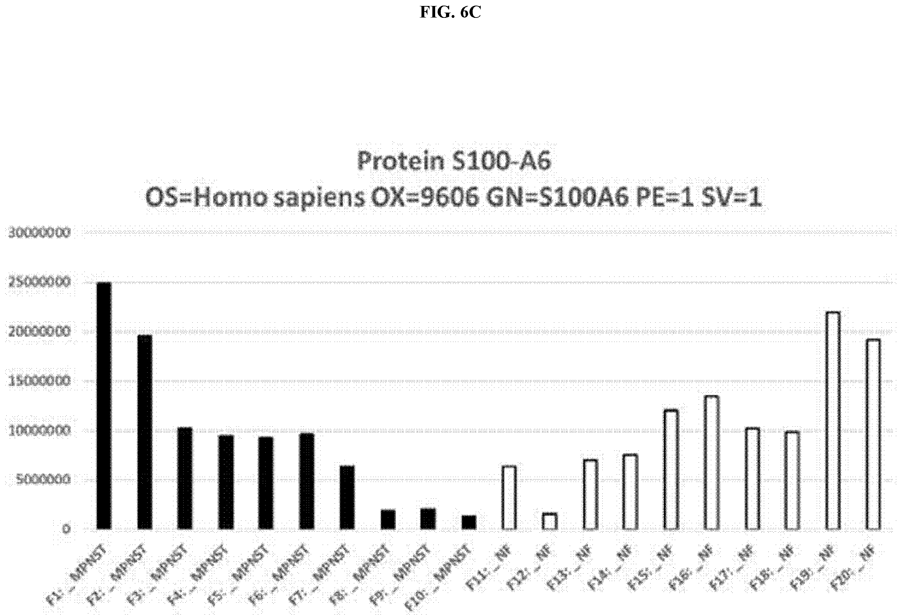

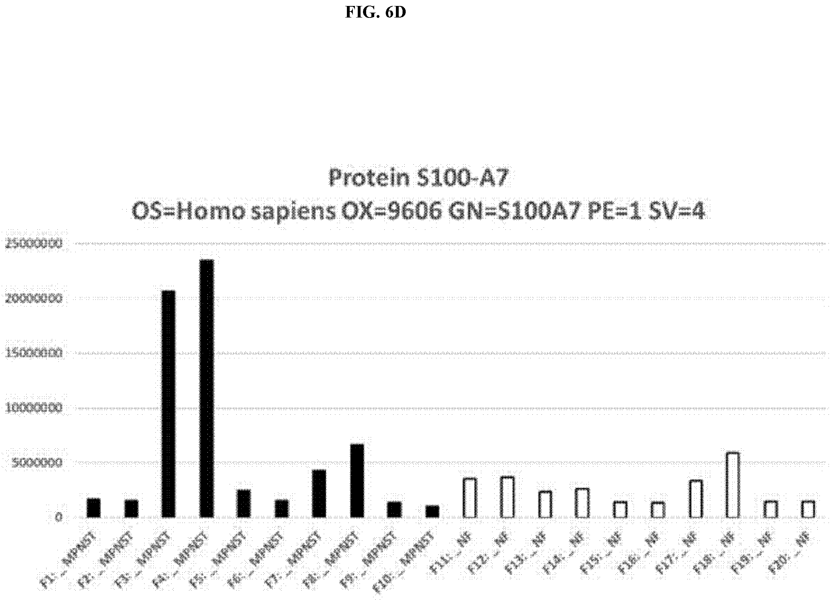

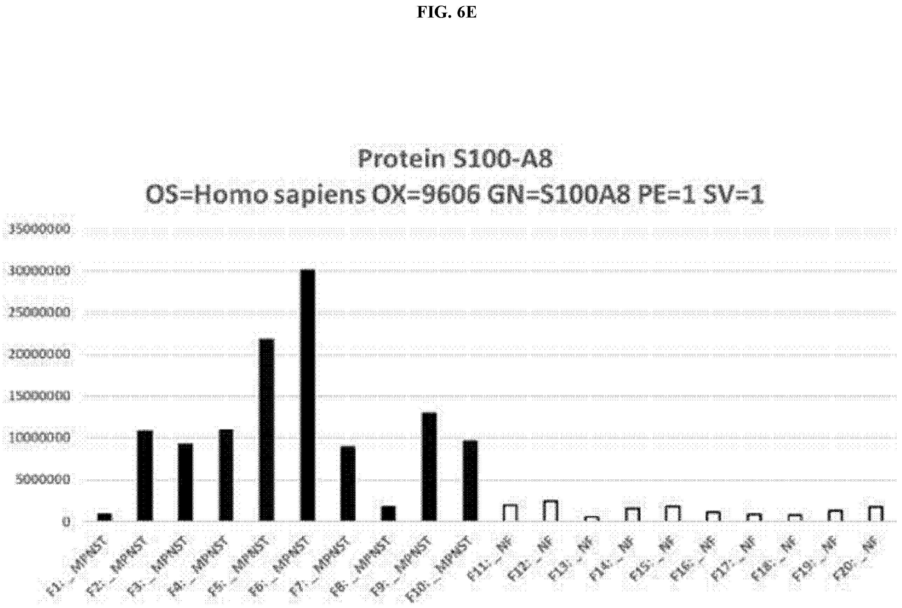

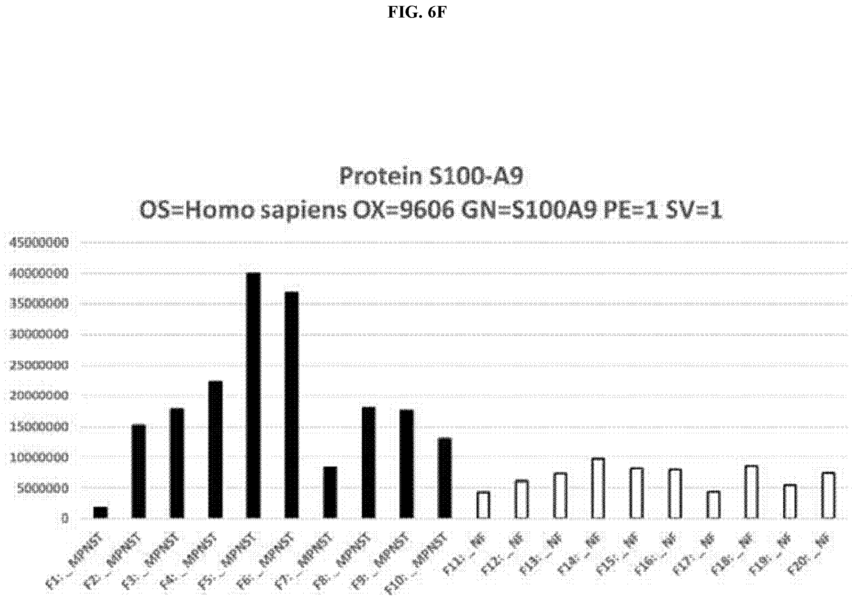

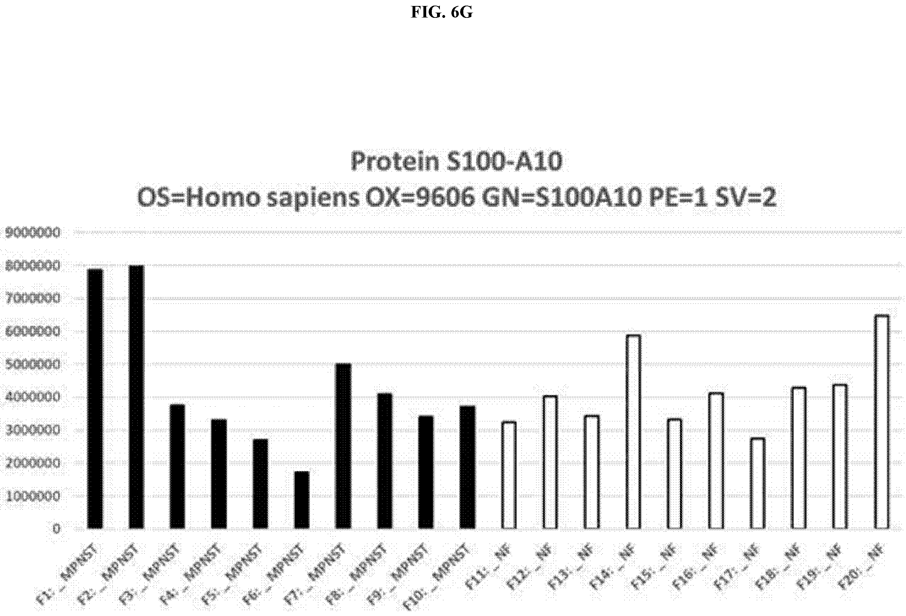

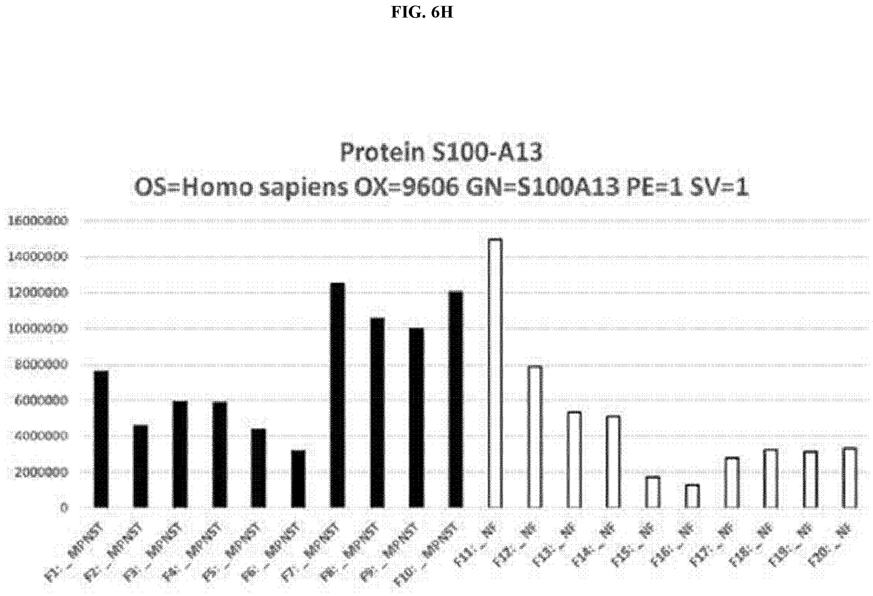

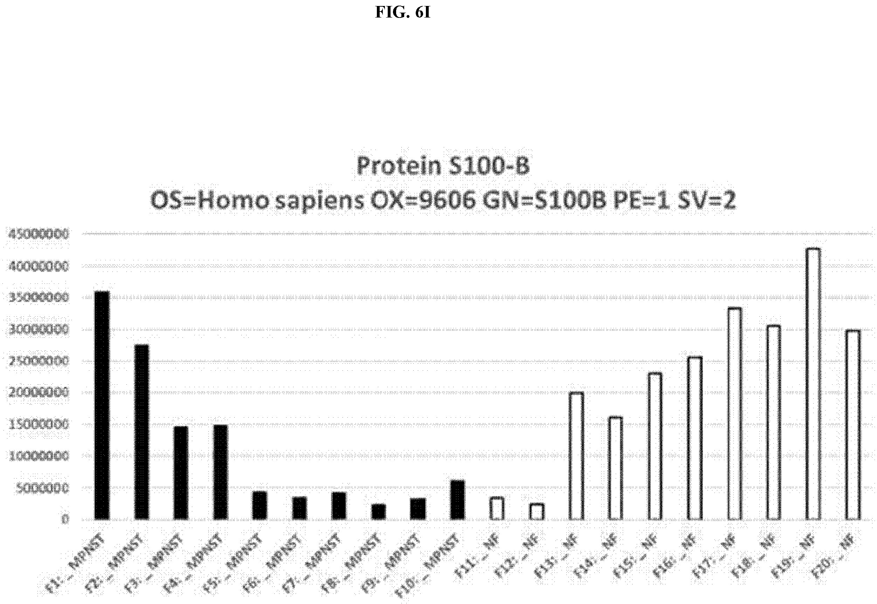

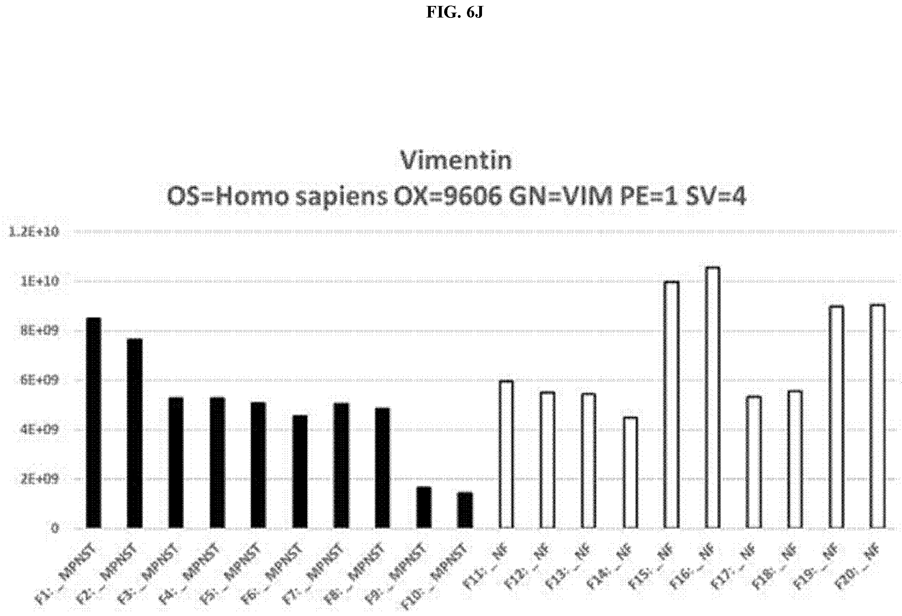

FIGS. 6A to 6J are graphs showing the result of investigating the expression patterns of S-100 protein isoforms and vimentin in NF and MPNST tissue, in which FIG. 6A shows the result for S100-A1 protein, FIG. 6B shows the result for S100-A4 protein, FIG. 6C shows the result for S100-A6 protein, FIG. 6D shows the result for S100-A7 protein, FIG. 6E shows the result for S100-A8 protein, FIG. 6F shows the result for S100-A9 protein, FIG. 6G shows the result for S100-A10 protein, FIG. 6H shows the result for S100-A13 protein, FIG. 6I shows the result for S100-B protein, and FIG. 6J shows the result for vimentin (F1-F10 indicates a patient's MPNST site sample, and F11-F20 indicates a patient's benign NF site sample. Specifically, F1 and F2 are MPNST site samples of Patient 1; F3 and F4 are MPNST site samples of Patient 2; F5 and F6 are MPNST site samples of Patient 3; F7 and F8 are MPNST site samples of Patient 4; F9 and F10 are MPNST site samples of Patient 5; F11 and F12 are benign NF site samples of Patient 1; F13 and F14 are benign NF site samples of Patient 2; F15 and F16 are benign NF site samples of Patient 3; F17 and F18 are benign NF site samples of Patient 4; and F19 and F20 are benign NF site samples of Patient 5 (refer to Table 3))
FIG. 7 is a graph showing the result of investigating the expression patterns of endosialin in benign NF and MPNST tissue.
FIG. 8 is a graph showing the result of investigating the expression patterns of SPARC-like protein 1 in benign NF and MPNST tissue.
FIG. 9 is a graph showing the result of investigating the expression patterns of neutrophil collagenase in benign NF and MPNST tissue.
FIGS. 10A to 10C show the results of are hierarchical cluster analysis of proteomes whose expression has changed in benign NF and MPNST tissue, in which FIG. 10A is a diagram visualizing the entire results, FIG. 10B shows the cluster of a protein with increased expression in MPNST, and FIG. 10C shows the cluster of a protein with decreased expression in MPNST.
FIG. 11 shows the result of gene ontology analysis for MPNST-specific proteins.
FIG. 12 shows the result of ingenuity pathway analysis (IPA) for MPNST-specific proteins.

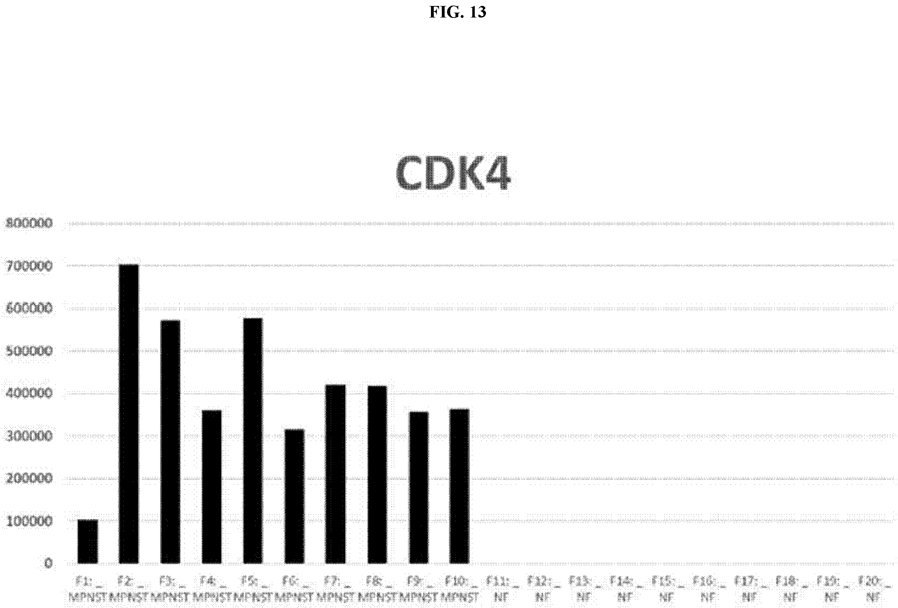

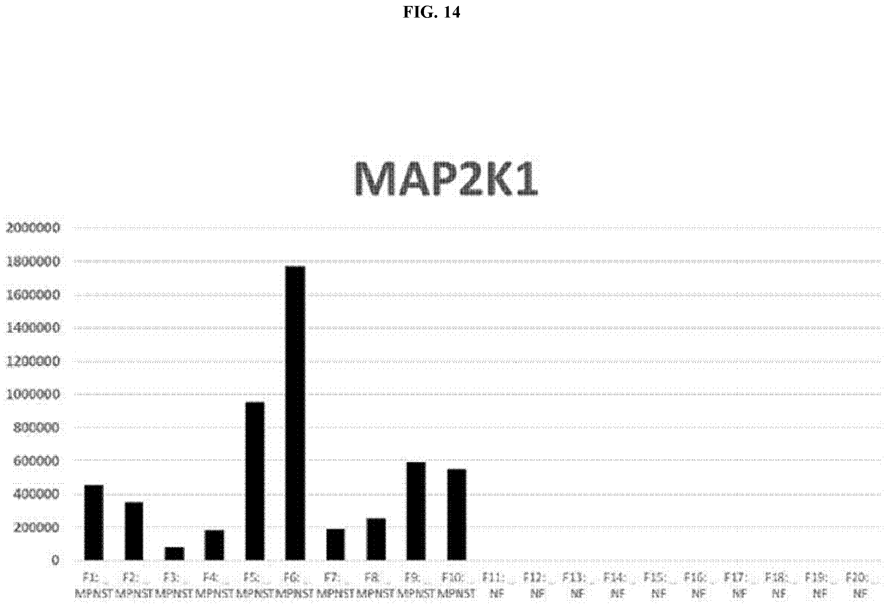

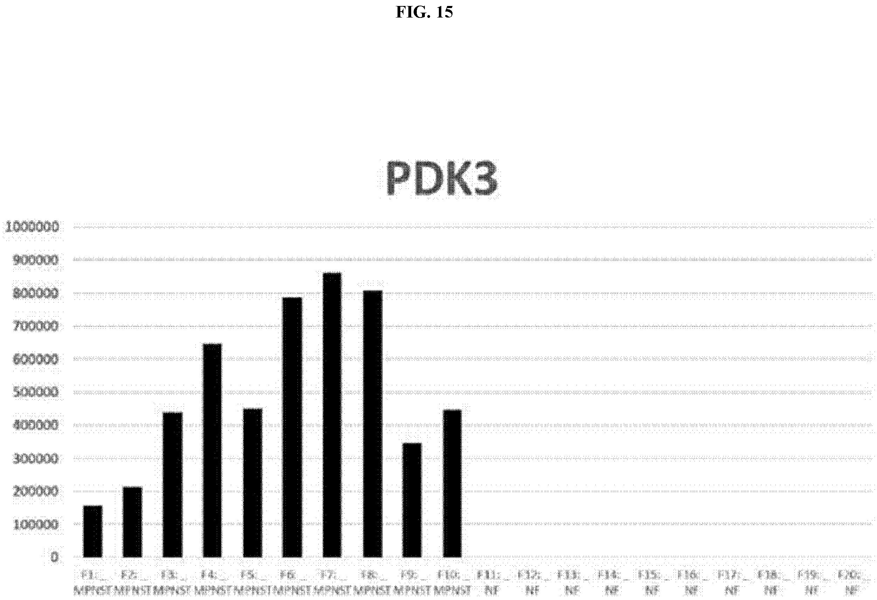

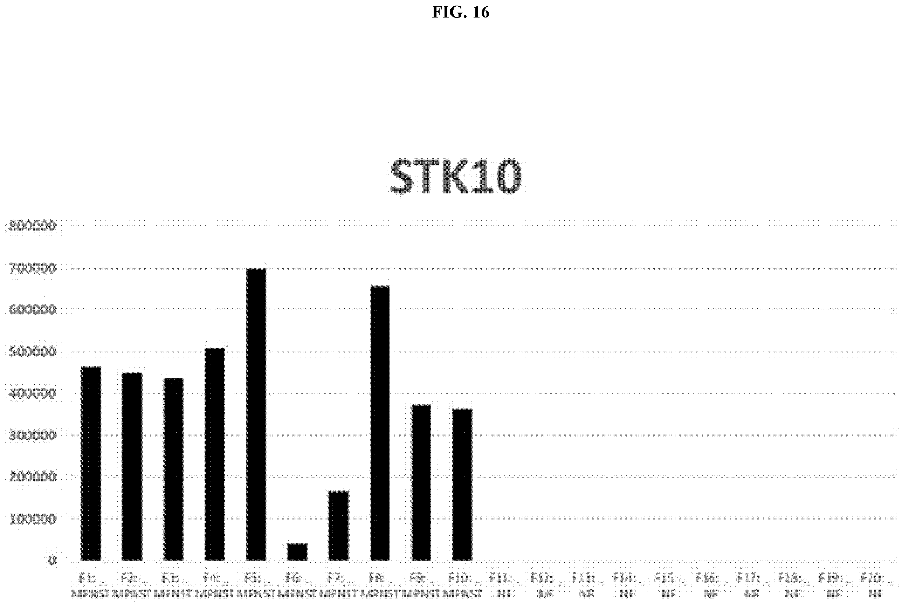

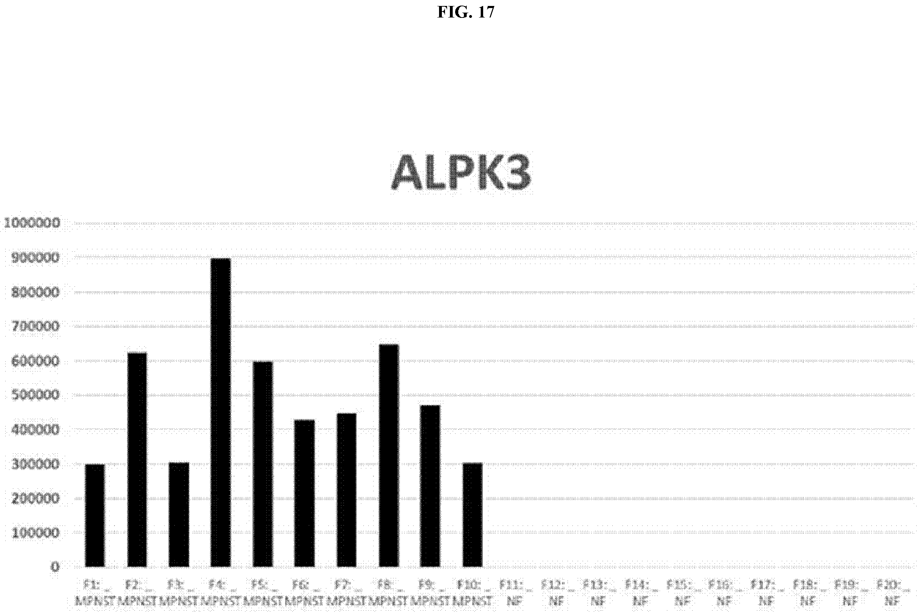

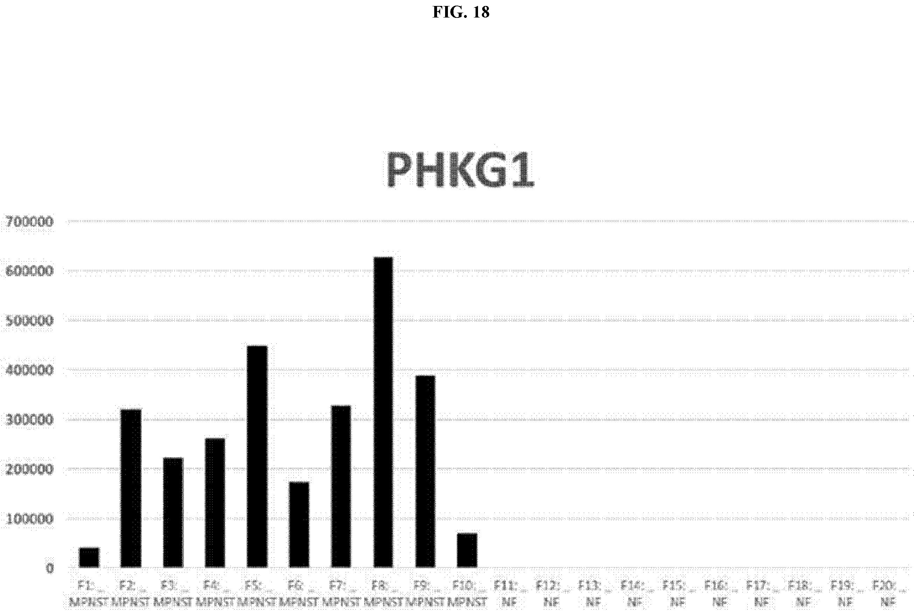

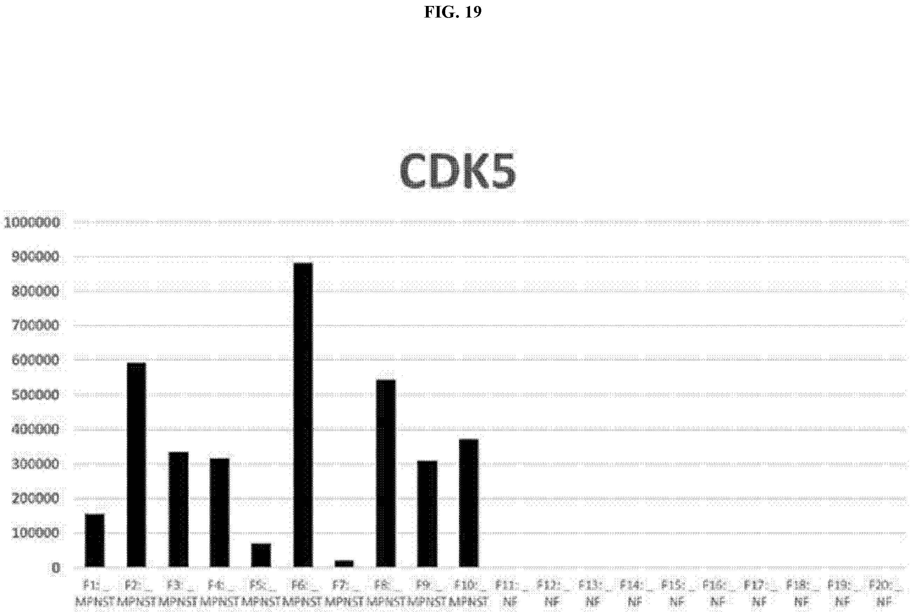

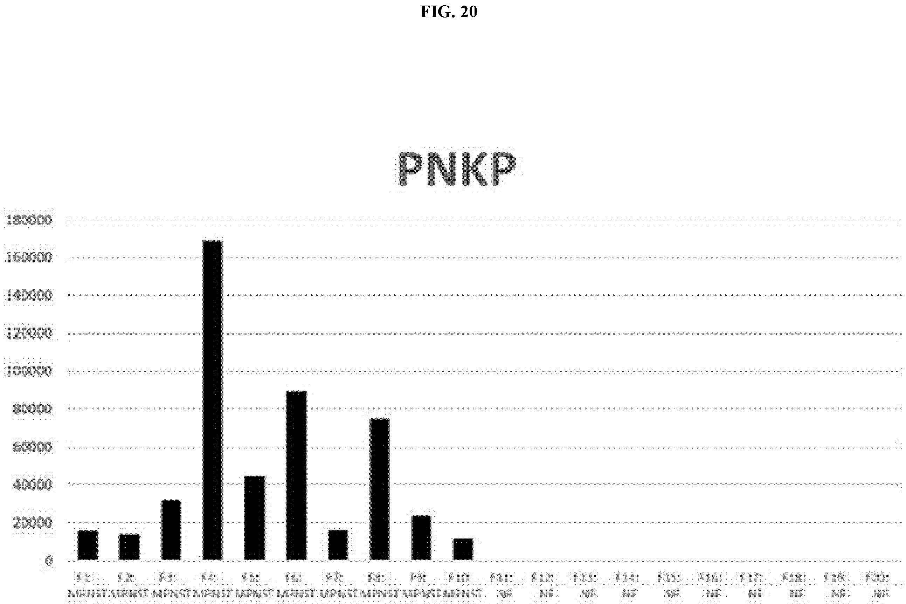

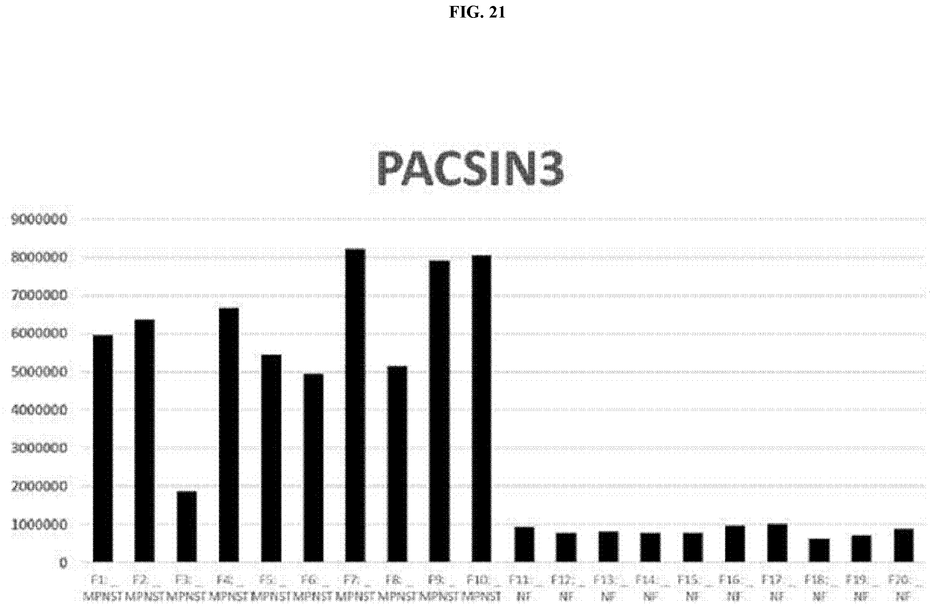

FIGS. 13 to 21 are graphs of comparative analyses of the abundance of kinases with specifically increased expression or activity in MPNST compared to benign NF, in which FIG. 13 shows the result of the abundance of CDK4, FIG. 14 shows the result of the abundance of MAP2K1, FIG. 15 shows the result of the abundance of PDK3, FIG. 16 shows the result of the abundance of STK10, FIG. 17 shows the result of the abundance of ALPK3, FIG. 18 shows the result of the abundance of PHKG1, FIG. 19 shows the result of the abundance of CDK5, FIG. 20 shows the result of the abundance of PNKP, and FIG. 21 shows the result of the abundance of PACSIN3.
FIG. 22 shows the immunohistochemistry (IHC) result for MMP8 protein (neutrophil collagenase) in benign NF and MPNST tissue (A1 and B1 indicate NF tissue, and A2 and B2 indicate MPNST tissue).
FIG. 23 shows the enzyme-linked immunosorbent assay (ELISA) result of analyzing the difference in MMP8 protein (neutrophil collagenase) expression in the presence or absence of tumor removal using an MPNST patient's blood.
FIG. 24 shows the immunohistochemistry (IHC) result for the ILK signaling pathway, actin-cytoskeleton signaling pathway, and kinases with specifically increased expression or activity in MPNST, compared to benign NF (A1 to J1 indicate NF tissue, and A2 to J2 indicate MPNST tissue).

### [Best Mode]

The present invention relates to a method of screening a plurality of biomarkers for differential diagnosis of the case in which neurofibromatosis type I (NF-1) transitions to malignant peripheral nerve sheath tumor (MPNST) and an MPNST therapeutic agent that targets an activated signaling pathway that is specifically stimulated by MPNST or a kinase whose expression is increased, and as a result of intensive research to discover an MPNST-specific diagnosis marker and a novel therapeutic target, the present inventors found a plurality of proteins and signaling pathways disclosed herein, and thus completed the present invention.

Hereinafter, the present invention will be described in detail.

The present invention relates to a composition for differential diagnosis of MPNST, which includes an agent measuring the expression level(s) of one or more proteins selected from the group consisting of the proteins disclosed in Table 1 below or the expression level of mRNA thereof as an active ingredient.

**[Table 1]**

| UniProt Accession No. | Gene | Protein | SEQ ID NO: |
|---|---|---|---|
| Q9HCU0 | CD248 | Endosialin | 1 |
| Q14515 | SPARCL1 | SPARC-like protein 1 | 2 |
| P22894 | MMP8 | Neutrophil collagenase | 3 |

In one embodiment of the present invention, through proteomic analysis using MPNST and benign NF tissue, proteins specific for MPNSTs were identified, and among these proteins, three types of proteins - endosialin, SPARC-like protein 1 and neutrophil collagenase -, which can be detected in blood or urine, are selected and shown to be able to be used as a biomarker for MPNSTs (refer to Examples 2 to 4 and 6).

The endosialin may be encoded by the CD248 gene, consist of the amino acid sequence of SEQ ID NO: 1, and includes a protein isoform in which amino acids 1 to 324 are deleted from the amino acid sequence of SEQ ID NO: 1. The SPARC-like protein 1 may be encoded by the SPARCL1 gene and consist of the amino acid sequence of SEQ ID NO: 2, and include an protein isoform in which amino acids 1 to 125 are deleted from the amino acid sequence of SEQ ID NO: 2. In addition, the neutrophil collagenase may be encoded by the MMP8 gene and consist of the amino acid sequence of SEQ ID NO: 3.

The term "protein" used herein is interchangeably used with polypeptide or peptide, and refers to, for example, a polymer of amino acid residues as commonly found in proteins in their natural state. The term "mRNA" refers to RNA delivering genetic information (gene-specific base sequences) to ribosomes that specify an amino acid sequence from specific genes in protein synthesis.

The term "expression" used herein refers to the generation of a protein or nucleic acid in cells.

The term "differential diagnosis" used herein refers to distinguishing a particular disease or condition from others exhibiting similar symptoms. A differential diagnosis method is a systemic diagnosis method used to confirm the presence of conditions in which multiple alternatives are possible. Such a method is essentially a process of elimination or an information acquisition process that reduces the probability of a candidate state to a negligible level. This term is also used to refer to the determination of the transition from benign NF to MPNST.

In the present invention, the composition may differentially diagnose an MPNST from NF-1.

The term "neurofibromatosis type I (NF-1)" or "benign neurofibromatosis" used herein refers to a disease first reported by Fredrich von Recklinghausen in 1882 as a genetic disorder exhibiting various clinical symptoms in the skin, skeletal system, and nervous system. Neurofibromatosis type I has characteristic symptoms such as cafe-au-lait-spots on the skin, axillary freckling, inguinal freckling, multiple neurofibromas, Lisch nodules, which are small, melanocytic hamartomas on the iris, optic gliomas, and bone dysplasia. In addition, neurofibromatosis type I also has symptoms such as a short stature, scoliosis, and learning disabilities. Most tumors occurring in neurofibromatosis type I patients are benign, but in rare cases, may develop into a malignancy, and may occur in any part of the body where nerves are present. According to research, it is reported that 67% of neurofibromatosis type I cases are found before the age of 1 year, 25-90% of cases are accompanied by cafe-au-lait-spots, which are characteristic skin lesions, and a maximum of 16% of cases are reported to become malignant.

The term "malignant peripheral nerve sheath tumor (MPNST)" used herein is interchangeably used with "malignant neurilemmoma," and generally, occurs in the limbs and head and neck, and rarely in the abdominal cavity, pelvis, and genitourinary system. Generally, MPNST is associated with NF-I, the risk of having MPNST in neurofibromatosis patients during their lifetime is known to be approximately 10%, and MPNST is one of the incurable diseases with a very poor prognosis and high malignancy.

When the composition of the present invention is for measuring the expression level of a protein, the agent for measuring the expression level of a protein may be an antibody or aptamer specific for the protein, but the present invention is not limited thereto.

The term "antibody" used herein refers to a specific protein molecule directed against an antigenic site. For the purpose of the present invention, the antibody refers to an antibody specifically binding to a marker protein, and includes all of polyclonal antibodies, monoclonal antibodies, and recombinant antibodies. In addition, as long as it has an antigen-antibody binding property, a part of the entire antibody is also included in the antibody of the present invention, and all kinds of immunoglobulin antibodies specifically binding to the proteins of the present invention are included. For example, the antibody used herein includes a complete antibody form with two full-length light chains and two full-length light chains as well as functional fragments of the antibody, that is, Fab, F(ab'), F(ab')₂, and Fv, each having an antigen-binding function. Furthermore, the antibody of the present invention includes special antibodies such as humanized antibodies and chimeric antibodies and recombinant antibodies as long as they can specifically bind to the protein.

The term "aptamer" used herein is a material capable of specifically binding to an analyte to be detected in a sample, and refers to a single-stranded nucleic acid (DNA, RNA, or modified nucleic acid) with a stable tertiary structure in itself, and it can specifically confirm the presence of a target protein in the sample. The aptamer may be prepared by synthesizing an oligonucleotide by determining a sequence thereof with selective and high binding affinity to a target protein to be confirmed, and modifying the 5' end or the 3' end of the oligonucleotide with -SH, -COOH, -OH, or NH₂ to bind to a functional group of an aptamer chip according to a general method of preparing an aptamer, but the present invention is not limited thereto.

The composition of the present invention including an antibody specific for a protein listed in Table 1 may further include an agent required for a known method of detecting a protein, and may measure a protein expression level in a subject (a biological sample acquired from the subject in the present invention) without a known method of detecting a protein using the composition.

In addition, when the composition of the present invention is for measuring the expression level of mRNA, an agent for measuring the expression level of mRNA may be a primer set or probe specifically binding to mRNA, but the present invention is not limited thereto.

The term "primer" used herein refers to a nucleic acid sequence with a short free 3-end hydroxyl group, which can form a base pair with a complementary template and serve as a starting point for the replication of a template strand. The primer may initiate DNA synthesis in the presence of a reagent for polymerization (i.e., DNA polymerase or reverse transcriptase) and four different nucleoside triphosphates in an appropriate buffer solution at an appropriate temperature.

The term "probe" used herein refers to a nucleic acid fragment such as RNA or DNA, which is as short as several bases or as long as hundreds of bases, capable of specifically binding to mRNA, and may be labeled to confirm the presence or absence of specific mRNA. Probes may be manufactured in the form of an oligonucleotide probe, a single stranded DNA probe, a double stranded DNA probe, or an RNA probe. The selection of a suitable probe and hybridization conditions may be changed based on those known in the art.

The "primer" or "probe" may be chemically synthesized using a method of synthesizing a solid support such as phosphoramidite or other well-known methods. In addition, the primer or probe may be modified in various ways according to methods known in the art to the extent that hybridization with mRNA is not hindered. Examples of such modifications are methylation, capping, substitution of natural nucleotides with one or more homologs, and modifications between nucleotides, for example, uncharged linkers (e.g., methyl phosphonate, phosphotriester, phosphoramidate, carbamate, etc.) or charged linkers (e.g., phosphorothioate, phosphorodithioate, etc.), and binding of a fluorescent or enzymatic labeling material.

The composition of the present invention including a primer set or probe specific for the mRNA of a protein listed in Table 1 may further include an agent required for a known method of detecting RNA. By using the known method of detecting RNA using the composition of the present invention without limitation, the levels of mRNA of the protein markers in subjects may be measured.

In addition, in the present invention, since the nucleic acid sequence of a gene encoding mRNA of the protein is registered in a gene bank, based on the sequence, an antisense oligonucleotide, a primer pair, or a probe, which specifically amplifies a specific region of the gene, may be designed by one of ordinary skill in the art.

The antisense oligonucleotide may be chemically synthesized using a solid support such as phosphoramidite or other well-known methods, and includes all functional equivalents. In addition, the above description of the "primer" or "probe" may be equally applied hereto.

The term "functional equivalent" means both nucleotides and nucleic acid sequences of altered mutant sequences and, including, for example, one or more substitutions, deletions or additions in a reference sequence, and the net effect that does not result in multiple functional dissimilarities between the reference sequence and a subject sequence. Conventionally, such a substantially equivalent sequence varies by only approximately 35% (i.e., the number of substitutions, additions or deletions of residues in a substantially equivalent sequence is approximately 0.35 or less when compared with the corresponding reference sequence and divided by the total number remaining in the substantially equivalent sequence) from the reference sequence, and examples thereof vary as listed in the specification. Such a sequence has 65% sequence identity with the listed sequences. Substantially equivalent sequences according to the present invention, for example, mutated amino acid sequences have preferably at least 80% sequence identity, and more preferably, at least 90% sequence identity compared to the listed amino acid sequences. Nucleotide sequences of the present invention, which are substantially equivalent, may have, for example, a lower percentage of sequence identity considering the redundancy or degeneracy of the genetic code. The nucleotide sequences preferably have at least approximately 65% sequence identity, more preferably, approximately 75% sequence identity, and most preferably, approximately 95% sequence identity. For the purpose of the present invention, sequences having substantially equivalent biological activity and substantially equivalent synthetic characteristics are considered substantially equivalent. To determine an equivalent, the truncation of a mature sequence (for example, through a mutation producing a spurious stop codon) must be neglected.

As another aspect of the present invention, the present invention provides a kit for differential diagnosis of MPNSTs, including the composition according to the present invention.

In the present invention, the kit may include instructions for determining a case as MPNST when the expression of one or more proteins selected from the group consisting of endosialin, SPARC-like protein 1, and neutrophil collagenase or the expression of mRNA thereof is detected, or when the expression level thereof is higher than that of a benign neurofibromatosis sample.

The kit of the present invention may include an antibody recognizing a target protein as a marker or a primer set or probe recognizing mRNA as a marker as well as a composition, solution or device, consisting of one or more types of components suitable for an analysis method.

As a specific aspect, the diagnostic kit may be a diagnostic kit that includes essential factors for carrying out a reverse transcription polymerase reaction. The reverse transcription polymerase reaction kit includes primer sets each specific for a marker gene. The primers are nucleotides having a specific sequence for each marker gene and are approximately 7 to 50 bp in length, and more preferably, approximately 10 to 30 bp in length. In addition, the diagnostic kit may include primers specific for the nucleic acid sequence of a control gene. Other than the above, the reverse transcriptase reaction kit may include test tubes or other suitable containers, reaction buffer solutions (with various pHs and magnesium concentrations), deoxyribonucleotides (dNTPs), enzymes such as Taq-polymerase and reverse transcriptase, DNAse and RNAse inhibitors, DEPC-water, and sterile water.

As another specific aspect, the diagnostic kit may be a diagnostic kit including essential factors required to use a DNA chip. The DNA chip kit may include a substrate to which cDNA or an oligonucleotide corresponding to a gene or its fragment is attached, and reagents, agents and enzymes for manufacturing fluorescence-labeled probes. In addition, the substrate may include cDNA or an oligonucleotide corresponding to a control gene or its fragment.

As still another aspect of the present invention, the present invention may provide a method of providing information required for differential diagnosis of MPNSTs, which includes measuring the expression level(s) of one or more proteins selected from the group consisting of endosialin, SPARC-like protein 1, and neutrophil collagenase, or the expression level of mRNA thereof in a biological sample isolated from a subject.

As yet another aspect of the present invention, the present invention provides a method for differential diagnosis of MPNST, which includes measuring the expression level(s) of one or more proteins selected from the group consisting of endosialin, SPARC-like protein 1 and neutrophil collagenase, or the expression level of mRNA thereof in a biological sample isolated from a subject.

The method for differential diagnosis of MPNST may rapidly and conveniently diagnose the transition from NF-I to MPNST in a body fluid sample (e.g., urine or plasma) in addition to generated lesion tissue using a plurality of biomarkers, through which an immediate response to MPNST is possible.

Therefore, the method for differential diagnosis of MPNST may be effectively used for treatment of NF- I.

In the present invention, the method may further include diagnosing a case as MPNST when the expression of one or more proteins selected from the group consisting of endosialin, SPARC-like protein 1, and neutrophil collagenase or the expression of mRNA thereof is detected, or when the expression level thereof is higher than that of a biological sample isolated from an NF-1 patient.

In yet another aspect of the present invention, the present invention may provide a method of treating MPNSTs, which includes the following steps:
(a) measuring the expression level(s) of one or more proteins selected from the group consisting of endosialin, SPARC-like protein 1, and neutrophil collagenase, or the expression level of mRNA thereof in a biological sample isolated from a subject;
(b) diagnosing a case as MPNST when the expression of one or more proteins selected from the group consisting of endosialin, SPARC-like protein 1, and neutrophil collagenase or the expression of mRNA thereof is detected, or when the expression level thereof is higher than that of a biological sample isolated from an NF-1 patient; and
(c) treating the MPNST diagnosed in (b).

In the present invention, the treating of the MPNST may use a method such as chemotherapy, radiation therapy, surgery, or biological therapy.

In the present invention, "chemotherapy" means the action of using a chemical for the treatment of a specific disease and all of the drugs used at that time.

In the present invention, the drug may be one or more anticancer agents selected from the group consisting of, for example, paclitaxel, doxorubicin, 5-fluorouracil, cisplatin, imatinib, carboplatin, oxaliplatin, tegafur, irinotecan, docetaxel, cyclophosphamide, cemcitabine, ifosfamide, mitomycin C, vincristine, etoposide, methotrexate, topotecan, tamoxifen, vinorelbine, camptothecin, danuorubicin, chlorambucil, bryostatin-1, calicheamicin, mayatansine, levamisole, DNA recombinant interferon alfa-2a, mitoxantrone, nimustine, interferon alfa-2a, doxifluridine, formestane, leuprolide acetate, megestrol acetate, carmofur, teniposide, bleomycin, carmustine, heptaplatin, exemestane, anastrozole, estramustine, capecitabine, goserelin acetate, polysaccharide potassium, medroxypogesterone acetate, epirubicin, letrozole, pirarubicin, topotecan, altretamine, toremifene citrate, BCNU, taxotere, actinomycin D, synthetic analogues thereof, and materials modified or exhibiting the same efficacy, but the present invention is not limited thereto.

In the present invention, the drug may be an antagonist against a protein listed in Table 2 below, and for example, an inhibitor of the activity of the signaling protein listed in Table 2, such as one or more selected from the group consisting of selumetinib, alvocidib, purvalanol, palbociclib, ribociclib, abemaciclib, fostamatinib, binimetinib, radicicol, AZD-8330, dihydrolipoic acid, alsterpaullone, hymenialdisine, indirubin-3'-monoxime, olomoucine, SU9516, and 6-phenyl[5H]pyrrolo[2,3-B]pyrazine, but the present invention is not limited thereto.

In the present invention, "radiation therapy" means exposing a patient to high-energy radiation, including, but not limited to, x-rays, gamma rays, and neutrons. This type of therapy may include external light therapy, internal radiation therapy, implant radiation, brachytherapy, and systemic radiation therapy, but the present invention is not limited thereto.

In the present invention, "surgery" includes any therapeutic or diagnostic procedure involving the methodical action of hands or hands together with a device with respect to the body of an individual to achieve a curative, therapeutic or diagnostic effect.

In the present invention, "biological therapy" means a therapeutic method that directly/indirectly uses the immune system of the human body by using a biological agent containing a material derived from an organism or an organism, and the biological agent includes vaccines whose potency and safety cannot be evaluated only by a physical or chemical test, allergens, antigens, hormones, cytokines, enzymes, blood and plasma, immune sera, monoclonal antibodies, fermentation products, antitoxins, and laboratory diagnostics.

In the present invention, the biological agent may be one or more selected from the group consisting of, for example, adalimumab, alemtuzumab, bevacizumab, cetuximab, daratumumab, panitumumab, rituximab, trastuzumab, pertuzumab, ipilimumab, nivolumab, pembrolizumab, atezolizumab, durvalumab, avelumab, tocilizumab, sarilumab, satralizumab, and siltuximab, but the present invention is not limited thereto.

The term "detection" used herein means both measuring and confirming the presence (expression) of a target material (marker protein in the present invention), or measuring and confirming a change in expression level of a target material. In the same context, the measuring of the expression level of the protein means measuring whether expression occurs or not (i.e., measuring the presence or absence of expression), or measuring the levels of qualitative and quantitative changes of the protein. The measurement may be performed by both a qualitative method (analysis) and a quantitative method without limitation. In the measurement of a protein level, types of qualitative and quantitative methods are well known in the art, and include the experimental methods described herein. A specific protein level comparison method for each method is well known in the art. Accordingly, the detection of a desired protein includes detection of the presence of a protein listed in Table 1, or confirmation of an increase (up-regulation) or a decrease (down-regulation) of the expression level of the protein.

In addition, the term "differential diagnosis" includes, in addition to the above-described meaning, determining the susceptibility of a subject to a specific disease or disorder, determining whether a subject currently has a specific disease or disorder, determining the prognosis of a subject with a specific disease or disorder, or therametrics (e.g., for monitoring the state of a subject to provide information on therapeutic efficiency).

In the present invention, the subject may be a patient suspected of having MPNST, or a patient diagnosed with NF-1.

In the present invention, the biological sample may be, but is not limited to, any one that is collected from a subject for whom the differential diagnosis of MPNSTs is to be performed, and may include, for example, tissue, cells, blood, plasma, sera, saliva, nasal fluid, sputum, synovial fluid, amniotic fluid, ascites, cervical secretion, vaginal secretion, urine, and cerebrospinal fluid, which are obtained by biopsy, and also include a microneedle aspiration specimen and microneedle washing fluid. Preferably, the measurement may be performed on one or more selected from the group consisting of a biological liquid sample, for example, blood, whole blood, plasma, urine, tissue, cells, an organ, bone marrow, microneedle aspiration specimen, microneedle washing fluid, a core needle biopsy specimen, and saliva. The present invention can diagnose MPNST from a body liquid sample (e.g., urine or plasma) in addition to lesion tissue in which the disease has occurred, and has great technical significance because it increases the effectiveness, speed, convenience and safety of diagnosis.

The biological sample may be pretreated before being used in detection or diagnosis. For example, this may include homogenization, filtration, distillation, extraction, concentration, inactivation of an interfering component, and the addition of a reagent. The sample may be prepared to increase the detection sensitivity of a protein marker, and for example, a serum sample collected from a patient may be pretreated by using anion exchange chromatography, affinity chromatography, size exclusion chromatography, liquid chromatography, sequential extraction, or gel electrophoresis.

The measurement of a protein expression level may be performed by any method of measuring protein expression known in the art without particular limitation, and may be, for example, one or more selected from the group consisting of western blotting, enzyme linked immunosorbent assay (ELISA), radioimmunoassay (RIA), radioimmunodiffusion, Ouchterlony immunodiffusion, Rocket immunoelectrophoresis, tissue immunostaining, immunoprecipitation assay, complement fixation assay, fluorescence-activated cell sorting (FACS) and a protein chip.

The measurement of an mRNA expression level may be performed by one or more selected from the group consisting of PCR, RNase protection assay, northern blotting, southern blotting, in situ hybridization, and a DNA chip according to a method of measuring gene expression known in the art, but the present invention is not limited thereto.

The term "analysis" used herein preferably refers to "measurement," qualitative analysis may refer to measurement and confirmation of the presence of a target material, and quantitative analysis may refer to measurement and confirmation of a change in expression level or amount of a target material. In the present invention, analysis or measurement may be performed by both qualitative and quantitative methods without limitation, and preferably, by a quantitative method.

Meanwhile, it is known that the pathogenesis of an MPNST in NF-1 involves abnormal activation, for example, the complete loss of intratumoral NF-1, the excessive activation of a RAS-MAPT signal, the loss of the CDKN2A gene, the loss of constituent genes, SUZ 12 and EED, of polycomb repressive complex 2 (PRC2), the loss of tumor suppressor gene TP53, the abnormal activation of the PI3K-AKT-mTOR signaling pathway, epidermal growth factor receptor (EGFR), platelet-derived growth factor receptor (PDGF-R), or vascular endothelial growth factor receptor (VEGFR). Based on this, various clinical studies on MPNSTs have been conducted, there was no study in which a significant therapeutic effect was exhibited. Accordingly, there is a need for searching for novel treatment targets and candidate materials using a novel approach.

In the present invention, according to such a need, four signaling pathways specifically activated in MPNSTs - the ILK signaling pathway, the actin-cytoskeleton signaling pathway, the RhoGDI signaling pathway, the TGF-β signaling pathway - and ten kinases with specifically increased expression or activity in MPNSTs - kinases encoded by CDK4, MAP2K1, PDK3, STK10, ALPK3, PHKG1, CDK5, PNKP, CFL1, and PACSIN3 genes - were discovered, and it was confirmed that they can be utilized as a new treatment target for MPNSTs (refer to Examples 5 and 7).

Accordingly, as yet another aspect of the present invention, the present invention may provide a pharmaceutical composition for preventing or treating MPNSTs, which includes an inhibitor of the activity of one or more proteins involved in signaling pathways selected from the group consisting of the TGF-β signaling pathway, the ILK signaling pathway, the actin-cytoskeleton signaling pathway, and the RhoGDI signaling pathway, or an inhibitor of the expression of a gene encoding the protein as an active ingredient.

As yet another aspect of the present invention, the present invention may provide a health functional food composition for preventing or improving MPNSTs, which includes an inhibitor of the activity of one or more proteins involved in signaling pathways selected from the group consisting of the TGF-β signaling pathway, the ILK signaling pathway, the actin-cytoskeleton signaling pathway, and the RhoGDI signaling pathway, or an inhibitor of the expression of a gene encoding the protein as an active ingredient.

The "TGF beta signaling (TGF-β) signaling protein" includes all proteins directly/indirectly involved in the TGF-β signaling pathway. For example, the TGF-β signaling protein is SMAD1, SMAD2, SMAD3, SMAD5, SMAD9, Thrombospondin-1, Rock, Cdc42/RAC, PAK, LIMK, PI3K, Ras, Raf, MEK1/2, ERK1/2, JNK, P38, Cofilin, mTOR, or AKT.

The "integrin-linked kinase (ILK) signaling protein" includes all proteins directly/indirectly involved in the ILK signaling pathway. For example, the ILK signaling protein is mTOR, AKT, VEGF, NF-κGSK3β, SNAIL, AP-1, MMP9, JAK2, Cdc42/RAC, SNAI1, JNK, PI3K, AP-1, C-Jun, HIF1α, COX2, or β-Catenin.

The actin-cytoskeleton signaling protein may include all proteins directly/indirectly involved in the actin-cytoskeleton signaling pathway. For example, the actin-cytoskeleton signaling protein is PI3K, CaM, PKA, Rho, ROCK, Ras, Rac, Paxillin, FAK, Cofilin, Cortactin, WAVE, PIX, TIAM1, Vav, PAK1, GEF, VASP, MEKK, MEK, MLCK, MLC, LIMK, ADF, CapZ, Hsp90, Gelsolin, ADF, Filamin, Profilin, RhoGEF, Myosin, ACTN, Vinculin, ACTN, PFN, MEK1/2, or ERK1/2.

The "Rho GDP-dissociation inhibitor (RhoGDI) signaling protein" includes all proteins directly/indirectly involved in the RhoGDI signaling pathway. For example, the RhoGDI signaling protein is MKK4/7, JNK, C-Jun, PARP, ROCK1, ROCK2, Cdc42, eNOS, Tau, MLCP, MLC, CRMP2, Adducin, LIMK, PKN, BORGs, Citron, WASP, MRCK, PI3K, PAK, PAR6, or PAR3.

In the present invention, the signaling protein may be a kinase, and the kinase may be one or more kinases selected from the group consisting of proteins listed in Table 2 below, but the present invention is not limited thereto.

In the present invention, among the kinases in Table 2 below, "CDK4," "STLK10," and "PHKG1" may be involved in the ILK signaling pathway; "CDK5" may be involved in the actin-cytoskeleton signaling pathway and the RhoGDI signaling pathway; "MAP2K1" and "CFL1" may be involved in the actin-cytoskeleton signaling pathway and the TGF-β signaling pathway; "ALPK3" may be involved in heart development; "PNKP" may be involved in DNA damage and repair; "PDK3" may be involved in pyruvate metabolism and the citric acid (TCA) cycle; and "PACSIN3" may be involved in the actin-cytoskeleton signaling pathway, but the present invention is not limited thereto.

**[Table 2]**

| UniProt Accession No. | Gene | Protein | SEQ ID NO. |
|---|---|---|---|
| P11802 | CDK4 | Cyclin-dependent kinase 4 | 4 |
| 094804 | STK10 | Serine/threonine-protein kinase 10 | 5 |
| Q00535 | CDK5 | Cyclin-dependent-like kinase 5 | 6 |
| Q02750 | MAP2K1 | Dual specificity mitogen-activated protein Kinase kinase1 | 7 |
| Q96L96 | ALPK3 | Alpha-protein kinase 3 | 8 |
| Q9T60 | PNKP | Bifunctional polynucleotide phosphatase/kinase | 9 |
| Q15120 | PDK3 | [Pyruvate dehydrogenase (acetyl-transferring)] kinase isozyme 3, mitochondrial | 10 |
| Q16816 | PHKG1 | Phosphorylase b kinase gamma catalytic | 11 |
| Q9UKS6 | PACS 1N3 | chain, skeletal muscle/heart protein kinase C isoform and casein kinase substrate in neurons protein 3 | 12 |
| P23528 | CFL1 | Cofilin | 13 |

In the present invention, as it was confirmed that the proteins listed in Table 2 (including p-MEK) is specifically up-regulated in MPNSTs, materials that can suppress the proteins, for example, antagonists against the proteins may be used as MPNST therapeutic agents.

In the present invention, an inhibitor of the activity of the protein may be a peptide, an antibody, an aptamer, and a compound, which specifically binds to the protein, but the present invention is not limited thereto.

The definitions of the terms "antibody" and "aptamer" used herein are as described above.

In the present invention, the inhibitor of the activity of the signaling protein may be one or more selected from the group consisting of selumetinib, alvocidib, purvalanol, palbociclib, ribociclib, abemaciclib, fostamatinib, binimetinib, radicicol, AZD-8330, dihydrolipoic acid, alsterpaullone, hymenialdisine, indirubin-3'-monoxime, olomoucine, SU9516, and 6-phenyl[5H]pyrrolo[2,3-B]pyrazine, but the present invention is not limited thereto.

In the present invention, a gene expression inhibitor may be one or more selected from the group consisting of miRNA, siRNA, shRNA, a ribozyme, a DNAzyme, a peptide nucleic acid (PNA), and an antisense oligonucleotide, which complementarily bind to the mRNA of the gene, but the present invention is not limited thereto.

The term "antisense oligonucleotide" used herein encompasses nucleic acid-based molecules that have a sequence complementary to target mRNA, particularly, the seed sequence of mRNA and thus can form a duplex with mRNA. Accordingly, the term "antisense oligonucleotide" used herein may be described as "complementary nucleic acid-based inhibitor."

The term "complementary" used herein to describe an antisense oligonucleotide means that an antisense oligonucleotide is sufficiently complementary to be selectively hybridized to the mRNA target under predetermined hybridization or annealing conditions, and preferably, physiological conditions, and encompasses both substantially complementary and perfectly complementary, and preferably means perfectly complementary.

The antisense oligonucleotide in the present invention includes various molecules. The antisense oligonucleotide is a DNA or RNA molecule, and more preferably, an RNA molecule. Selectively, the antisense oligonucleotide used in the present invention may be a ribonucleotide (RNA), a deoxyribonucleotide (DNA), a 2'-O-modified oligonucleotide, a phosphorothioate-backbone deoxyribonucleotide, a peptide nucleic acid (PNA) or a locked nucleic acid (LNA). The 2'-O-modified oligonucleotide may be, for example, a 2'-O-alkyl oligonucleotide, a 2'-O-C1-3 alkyl oligonucleotide, or a 2'-O-C1-3 methyl oligonucleotide.

The terms "miRNA," "siRNA," and "shRNA" used herein refer to nucleic acid molecules that mainly bind to mRNA transcribed from a target gene to mediate RNA interfering or gene silencing, thereby suppressing the translation of mRNA. Since the miRNA, siRNA, and shRNA can suppress the expression of a target gene at the translation level, they can be used for an efficient gene knockdown method or gene therapy method.

The term "ribozyme" used herein may recognize a specific nucleotide sequence in a target RNA molecule and site-specifically digest it, thereby suppressing protein expression of the target gene.

The term "PNA" used herein refers to a nucleic acid mimic, for example, a DNA mimic, and here, the deoxyribose phosphate backbone is replaced with a pseudopeptide backbone, and only the four original nucleobases are maintained. The neutral backbone of a PNA is known to provide a hybrid specific for DNA and RNA under a low ionic strength condition, and can be used as an antisense or antigenic agent for sequence-specific regulation of gene expression by inducing transcriptional or translational repression or suppressing replication.

Meanwhile, a content of the inhibitor of the activity of the signaling protein or the inhibitor of the expression of a gene encoding the protein in the composition of the present invention can be appropriately adjusted depending on the symptoms of a disease, the progression of the symptoms, and the condition of a patient, and the content may be, for example, 0.0001 to 99.9 wt%, or 0.001 to 50 wt% based on the total weight of the composition, but the present invention is not limited thereto. The content ratio is a value based on the dry amount after removing a solvent.

The pharmaceutical composition according to the present invention may further include suitable carriers, excipients and diluents, which are conventionally used in the preparation of a pharmaceutical composition. The excipient may be, for example, one or more selected from the group consisting of a diluent, a binder, a disintegrant, a lubricant, an adsorbent, a humectant, a film-coating material, and a controlled-release additive.

The pharmaceutical composition according to the present invention may be formulated into a powder, a granule, a sustained-release granule, an enteric granule, a liquid, an ophthalmic solution, an elixir, an emulsion, a suspension, a spirit, a troche, aromatic water, a lemonade, a tablet, a sustained-release tablet, an enteric tablet, a sublingual tablet, a hard capsule, a soft capsule, a sustained-release capsule, an enteric capsule, a pill, a tincture, a soft extract, a dry extract, a fluid extract, an injection, a capsule, a perfusate, a plaster, a lotion, a paste, a spray, an inhalant, a patch, a sterile injection, or an external preparation such as an aerosol according to a conventional method, and the external preparation may be formulated in the form of a cream, a gel, a patch, a spray, an ointment, a plaster, a lotion, a liniment, a paste, or a cataplasma.

The carrier, excipient and diluent, which may be included in the pharmaceutical composition according to the present invention, may include lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil.

The pharmaceutical composition may be formulated with a diluent or an excipient such as a filler, a thickening agent, a binder, a wetting agent, a disintegrant, and a surfactant, which are commonly used.

As additives for a tablet, powder, granule, capsule, pill and troche according to the present invention, excipients such as corn starch, potato starch, wheat starch, lactose, sucrose, glucose, fructose, di-mannitol, precipitated calcium carbonate, synthetic aluminum silicate, calcium monohydrogen phosphate, calcium sulfate, sodium chloride, sodium bicarbonate, purified lanolin, microcrystalline cellulose, dextrin, sodium alginate, methylcellulose, carboxymethylcellulose, sodium carboxymethylcellulose, kaolin, urea, colloidal silica gel, hydroxypropyl starch, hydroxypropyl methyl cellulose, 1928, 2208, 2906, 2910, propylene glycol, casein, calcium lactate and Primojel; binders such as gelatin, gum arabic, ethanol, agar powder, cellulose acetate phthalate, carboxymethyl cellulose, carboxymethyl cellulose calcium, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch powder, hydroxypropylcellulose, hydroxypropylmethylcellulose, polyvinyl alcohol and polyvinylpyrrolidone; disintegrants such as hydroxypropylmethylcellulose, corn starch, agar powder, methylcellulose, bentonite, hydroxypropyl starch, sodium carboxymethylcellulose, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, sucrose, magnesium aluminum silicate, a di-sorbitol solution and light anhydrous silicic acid; and lubricants such as calcium stearate, magnesium stearate, stearic acid, hydrogenated vegetable oil, talc, limestone kaolin, petrolatum, sodium stearate, cacao butter, sodium salicylate, magnesium salicylate, polyethylene glycol 4000 and, 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, a higher fatty acid, a higher alcohol, silicone oil, paraffin oil, polyethylene glycol fatty acid ether, starch, sodium chloride, sodium acetate, sodium oleate, dileucine and light anhydrous silicic acid may be used.

As additives for liquid formulation according to the present invention, water, diluted hydrochloric acid, diluted sulfuric acid, sodium citrate, monostearate sucrose, polyoxyethylene sorbitol fatty acid esters (Tween esters), polyoxyethylene monoalkylethers, lanolin ethers, lanolin esters, acetic acid, hydrochloric acid, acetic acid, hydrochloric acid, aqueous ammonia, ammonium carbonate, potassium hydroxide, sodium hydroxide, prolamine, polyvinylpyrrolidone, ethyl cellulose, and sodium carboxymethylcellulose may be used.

For a syrup according to the present invention, a solution of white sugar, other sugars, or a sweetener may be used, and if necessary, a flavoring agent, a colorant, a preservative, a stabilizer, a suspending agent, an emulsifier, and a thickener may be used.

For an emulsion according to the present invention, purified water may be used, and if necessary, an emulsifier, a preservative, a stabilizer, and a fragrance may be used.

For a suspension according to the present invention, a suspending agent such as acacia, tragacanth, methylcellulose, carboxymethylcellulose, sodium carboxymethylcellulose, microcrystalline cellulose, sodium alginate, hydroxypropylmethylcellulose (HPMC), HPMC 1828, HPMC 2906, or HPMC 2910 may be used, and if necessary, a surfactant, a preservative, a stabilizer, a colorant, and a fragrance may be used.

An injection according to the present invention may include a solvent such as injectable sterile water, 0.9% sodium chloride for injection, Ringer's solution, a dextrose for injection, dextrose+sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid or benzene benzoate; a solubilizing agent such as sodium benzoate, sodium salicylate, sodium acetate, urea, urethane, monoethylacetamine, butazolidine, propylene glycol, Tween, nicotinamide, hexamine or dimethylacetamide; a buffer such as a weak acid and a salt thereof (acetic acid and sodium acetate), a weak base and a salt thereof (ammonia and ammonium acetate), an organic compound, a protein, albumin, peptone, or gums; an isotonic agent such as sodium chloride; a stabilizer such as sodium bisulfite (NaHSO₃), carbon dioxide gas, sodium metabisulfite (Na₂S₂O₃), sodium sulfite (Na₂SO₃), nitrogen gas (N₂) or ethylenediaminetetracetic acid; an antioxidant such as sodium bisulfide 0.1%, sodium formaldehyde sulfoxylate, thiourea, disodium ethylenediaminetetraacetate or acetone sodium bisulfite; a pain-relief agent such as benzyl alcohol, chlorobutanol, procaine hydrochloride, glucose or calcium gluconate; or a suspending agent such as sodium CMC, sodium alginate, Tween 80 or aluminum monostearate.

For a suppository according to the present invention, a base such as cacao butter, lanolin, Witepsol, polyethylene glycol, glycerogelatin, methyl cellulose, carboxymethylcellulose, a mixture of stearate and oleate, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydrokote SP, S-70-XXA, S-70-XX75 (S-70-XX95), Hydrokote 25, Hydrokote 711, Idropostal, Massa estrarium, A, AS, B, C, D, E, I, T), Mass-MF, Masupol, Masupol-15, neosuppostal-N, paramount-B, supposiro (OSI, OSIX, A, B, C, D, H, L), suppository base IV types (AB, B, A, BC, BBG, E, BGF, C, D, 299), Suppostal (N, Es), Wecoby (W, R, S, M ,Fs), or a Tegester triglyeride base (TG-95, MA, 57) may be used.

A solid formulation for oral administration may be a tablet, a pill, a powder, a granule or a capsule, and such a solid formulation may be prepared by mixing at least one excipient, for example, starch, calcium carbonate, sucrose, lactose and gelatin, with the active ingredient. Also, in addition to the simple excipient, lubricants such as magnesium stearate and talc may also be used.

As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative, may be included. A formulation for parenteral administration may be a sterilized aqueous solution, a non-aqueous solvent, a suspension, an emulsion, a lyophilizing agent or a suppository. As the non-aqueous solvent or suspension, propylene glycol, polyethylene glycol, a vegetable oil such as olive oil, or an injectable ester such as ethyl oleate may be used.

The pharmaceutical composition according to the present invention is administered at a pharmaceutically effective amount. The "pharmaceutically effective amount" used herein refers to an amount sufficient for treating a disease at a reasonable benefit/risk ratio applicable for medical treatment, and an effective dosage may be determined by parameters including the type and severity of a patient's disease, drug activity, sensitivity to a drug, administration time, an administration route and an excretion rate, the duration of treatment and drugs simultaneously used, and other parameters well known in the medical field.

The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without a side effect, and such a dose may be easily determined by one of ordinary skill in the art.

The pharmaceutical composition of the present invention may be administered to a subject via various routes. All administration routes may be expected, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined according to the type of drug as an active ingredient together with various parameters such as a disease to be treated, an administration route, a patient's age, sex, and body weight, and the severity of the disease.

The term "subject" used herein refers to a target in need of treatment, and more specifically, a mammal such as a human or a non-human primate, a mouse, a rat, a dog, a cat, a horse, or a cow.

In the present invention, "administration" means providing the given composition of the present invention to a subject by any suitable method.

In the present invention, "prevention" means all actions that inhibit or delay the onset of a target disease, and "treatment" means all actions involved in alleviating or beneficially changing symptoms of a target disease and symptoms of related metabolic abnormalities by administration of the pharmaceutical composition according to the present invention.

The term "health functional food" used herein refers to food manufactured and processed using raw materials or ingredients having useful functionalities for the human body in accordance with the Act for Health Functional Foods No. 6727, and means things eaten for useful effects on health, such as the control of nutrients or physiological action for the structure and function of the human body. The health functional food of the present invention may include conventional food additives, and the suitability as a food additive is judged by the specifications and standards for a corresponding item in accordance with the general rules and general test methods for the Korean Food Additives Code approved by the Ministry of Food and Drug Safety unless specified otherwise.

As yet another aspect of the present invention, the present invention may provide a method of screening a material for preventing, alleviating or treating MPNSTs, which includes: (a) bringing a candidate material into contact with isolated cells or tissue; (b) measuring the activity of one or more signaling protein(s) involved in signaling pathways selected from the group consisting of the TGF-β signaling pathway, the ILK signaling pathway, the actin-cytoskeleton signaling pathway, and the RhoGDI signaling pathway or an expression level of gene(s) encoding the protein(s) in the cells or tissue; and (c) determining the candidate as a material for preventing, alleviating or treating MPNSTs when the activity of the protein or the expression level of a gene encoding the protein in cells or tissue in contact with the candidate material is lower than that before contact with the candidate material.

In the present invention, the candidate material may include, but not limited to, a low molecular weight compound, a high molecular weight compound, a microbial culture or extract, a natural substance extract, a nucleic acid, a protein, a sugar, and a lipid, and may include any material that can reduce the activation of one or more signaling proteins involved in signaling pathways selected from the group consisting of the TGF-β signaling pathway, the ILK signaling pathway, the actin-cytoskeleton signaling pathway, and the RhoGDI signaling pathway or the expression level of a gene encoding the protein.

The nucleic acid may be selected from the group consisting of microRNA, siRNA, shRNA, antisense RNA, an aptamer, a locked nucleic acid (LNA), a peptide nucleic acid (PNA), and a morpholino, but the present invention is not limited thereto.

In the present invention, the signaling protein may be a kinase, and the kinase may be a kinase encoded by one or more genes selected from the group consisting of CDK4, STK10, CDK5, MAP2K1, ALPK3, PNKP, PDK3, PHKG1, CFL1, and PACSIN3, but the present invention is not limited thereto.

In the present invention, the isolated cells or tissue may be nerve cells or nerve tissue, but the present invention is not limited thereto.

The terms used in the present invention have been selected from general terms that are currently and widely used as much as possible while considering the functions of the present invention, but these may vary depending on the intentions of technicians skilled in the art, precedents, and the emergence of new technologies. In addition, in certain cases, there are also terms arbitrarily selected by the applicants, and in this case, their meanings will be described in detail in the description of the corresponding invention. Therefore, the terms used in the present invention should be defined based on the meanings of the terms and the content throughout the present invention, not simply based on the names of the terms used in the present invention.

Throughout the specification, when one part "includes" a component, it means that it may also include other components rather than excluding components unless specifically stated otherwise. The term "approximately" or "substantially" used herein are used at, or in proximity to, indicated numerical values when allowable manufacturing and material tolerances, which are inherent in the meanings, are provided. The above term is used to prevent the unfair use of the disclosures in which correct or absolute values are cited to help in understanding the present invention by unscrupulous infringers.

Throughout the specification, the term "combination thereof" included in the Markush-type expression refers to a mixture or combination of one or more selected from the group consisting of constituents described in the Markush-type expression, that is, one or more selected from the group consisting of the components.

### [Modes of the Invention]

Hereinafter, preferred examples are presented to better understand the present invention. However, the following examples are provided to more easily understand the present invention, and the content of the present invention is not limited by the following examples.

### [Examples]

### Example 1. Sample preparation and proteomic analysis

### 1.1. Sample preparation

MPNST tissue was collected from five patients diagnosed with neurofibromatosis type I (NF-1), and as a control, benign neurofibromatosis tissue collected from five patients was used. Information on the MPNST patients is shown in Table 3 below.

**[Table 3]**

| Patient | NF-1 germ cell mutation | MPNST origin | Pathological characteristics |
|---|---|---|---|
| 1 | c.7285C>T (p.R2429*) | Right femur | Spindle cell sarcoma, low grade, consistent with lower-grade MPNST, occurs in neurofibromas, 0.2 ×.9.1 × 5.2 cm, in muscles |
| 2 | c.1316T>C (p.L439P) | Right thigh | MPNST occurring in neurofibromas, high grade, 9.7 × 7 × 6.5 cm |
| 3 | c.6791dup (p.Y2264fs) | forearm | MPNST, FNCLCC grade, in the background of neurofibromas, 2 × 1.5 × 1.1 cm, forearm, dermal and subcutaneous |
| 4 | Not found | Pelvis | Spindle cell neoplasm, consistent with MPNST, low grade, in the background of neurofibromas, 12 × 7 x5 cm, left pubic area |
| 5 | c.2990G>C (p.R997T) | Pelvis | MPNST occurring in neurofibromas, FNCLCC grade, fragmented, 24 × 17 × 5.5 cm aggregate, pelvic cavity |

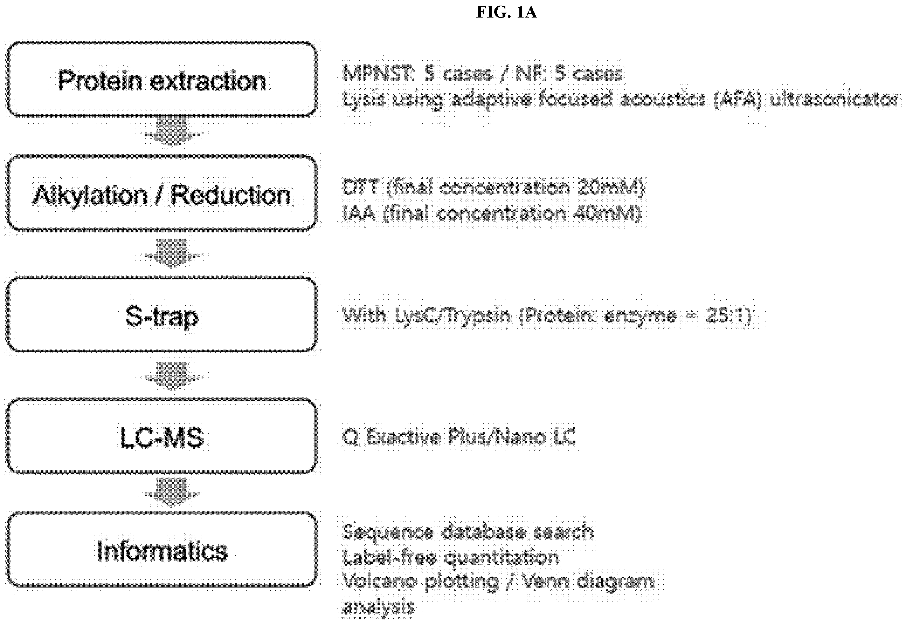

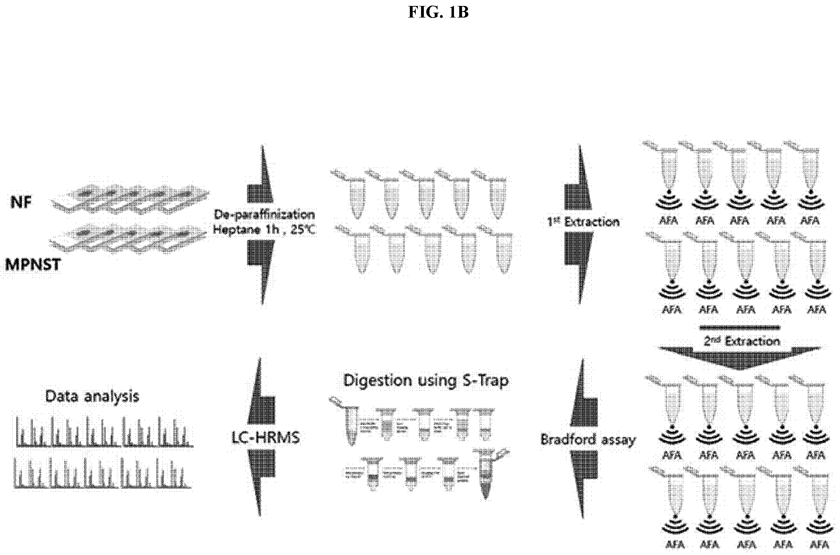

The collected tissue samples were fixed in formalin and then embedded in paraffin. To isolate proteomes, as shown in the flowcharts of FIGS. 1A and 1B, the formalin-fixed paraffin-embedded (FFPE) tissue slide was separated with a razor blade, placed in a tube, and deparaffinized with 200 µL of heptane at 25 °C for 1 hour. Subsequently, for disruption, extraction and homogenization of the sample, 5% SDS and 100 µL of 50 mM Triethylammonium biocarbonate buffer were added to lyse the sample using an adaptive focused acoustics (AFA) ultrasonicator. Afterward, the sample was digested using trypsin/LysC protease to prepare a peptide, and then a proteome was analyzed using LC-MS.

### 1.2. Proteomic analysis

Proteomic analysis was performed by LC-MS. As equipment for mass spectrometry (hereinafter, referred to as "MS"), Q-Exactive Plus BioPharm version (ThermoFisher, USA) was used, and as equipment for liquid chromatography (hereinafter, referred to as "LC"), an UltiMate^{™}3000 RSLCnano System (ThermoFisher, USA) was used. 5.0 µL of a sample was fed using an automatic sample feeding device and analyzed at a flow rate of 250 nl/min with an ion trap mass spectrometer coupled to NanoLC.

As a column used for LC, a fused silica capillary column C18 with an inner diameter of 75 µm and an outer diameter of 360 µm was used. The sample was analyzed for 200 minutes at a flow rate of 250 nl/min, and separation was performed with a concentration gradient from 5% solution B to 50% solution B (80% acetonitrile, 0.1% formic acid, 5% DMSO) for 90 minutes.

Parameters for MS were MS1 resolution 70000, MS1 maximum fill time of 20 msec, and a data dependent acquisition (DDA) method (top 10), and MS was performed under conditions including MS2 resolution 17500, MS2 maximum fill time of 100 msec, and auto gain control (AGC) 1e6.

### Example 2. Proteomic data analysis and identification of MPNSTspecific protein using the same

Proteomic data analysis was performed for the MPNST and benign neurofibromatosis (NF) tissue samples collected in Example 1.

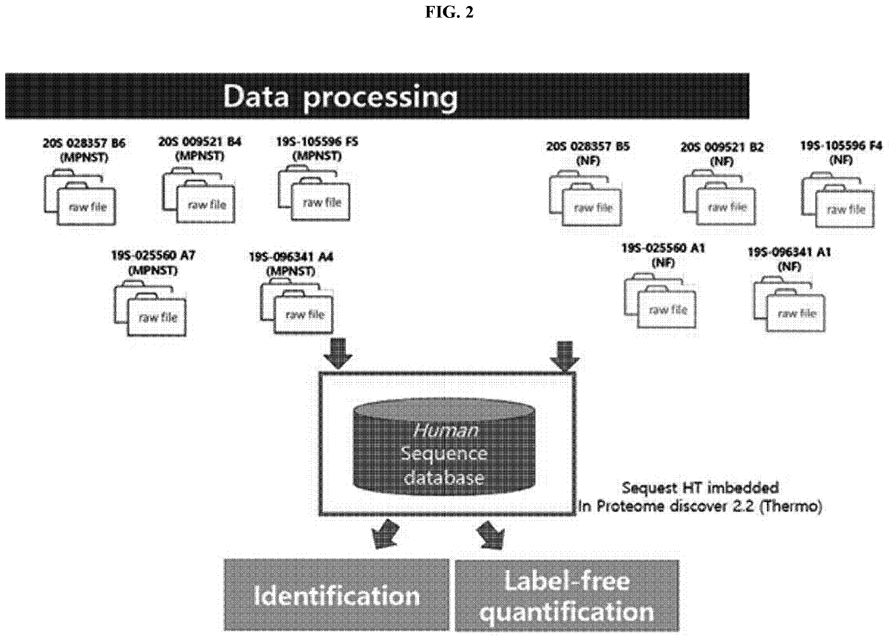

First, as shown in FIG. 2, each piece of raw data was analyzed using Proteome discover 2.2 software (Thermo, USA). The qualitative and quantitative information of the protein was extracted with a label-free quantitation algorithm using human proteome reference database provided by Uniprot. For qualitative analysis, a fixed modification condition among the peptide search conditions used carbamidomethylation on cysteine, and n-terminal acetylation and methionine oxidation were applied as variable modification conditions. The initial mass error of precursor ions was adjusted to 10 ppm, and the mass error for fragment ions was adjusted to 20 ppm, and when mapping a peptide to a protein, both a unique peptide and a razor peptide were used. For label-free quantification, the peak intensity of the unique peptide was used.

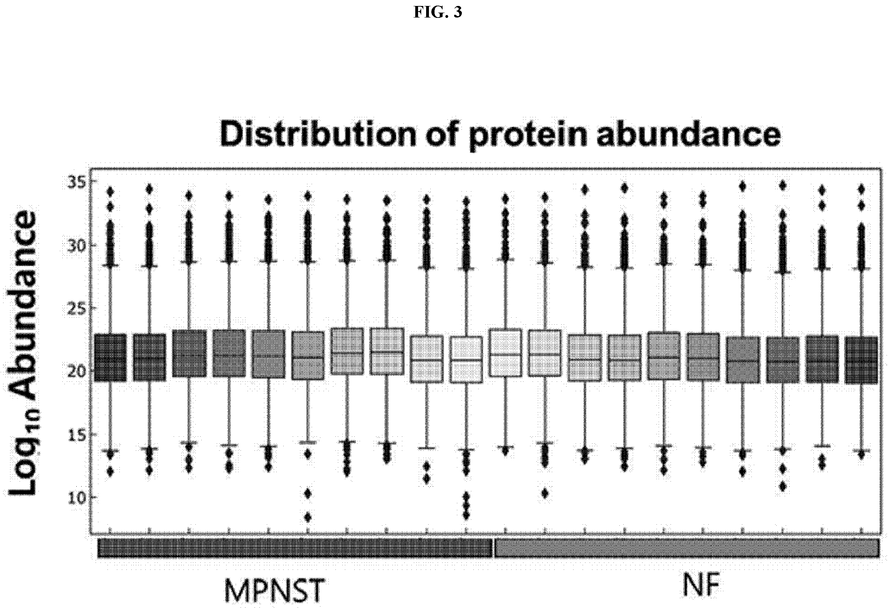

As a result, as shown in FIG. 3, it was confirmed that the distribution of quantitative values of the proteome obtained from each sample was uniform.

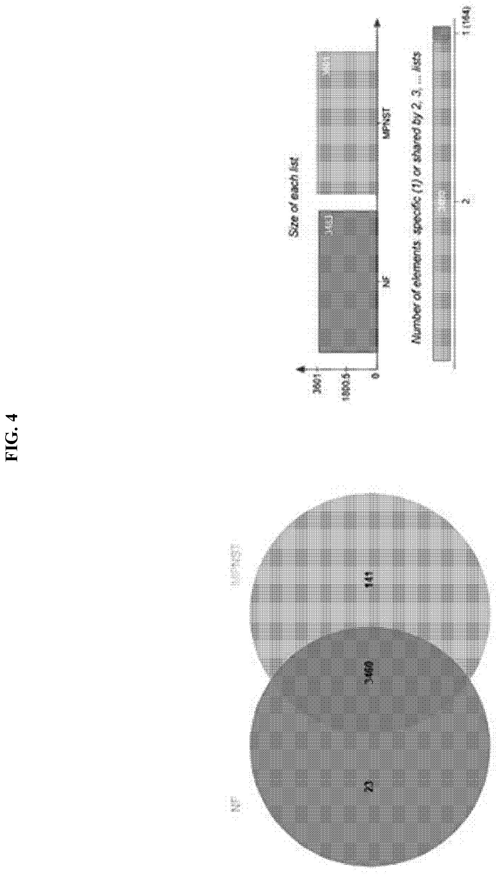

In addition, principal component analysis (PCA) was performed using quantitative proteomic data of the sample. As a result, as shown in FIG. 4, it was confirmed that two groups can be distinguished by the quantitative and qualitative differences in the proteome between the two groups, MPNST and NF. More specifically, referring to the right graph of FIG. 4, 3,483 proteins were identified in the benign NF tissue and 3,601 proteins in the MPNST tissue. 141 proteins were identified in only the MPNST tissue and not in the benign NF tissue, and 23 proteins were identified in the opposite case.

### Example 3. Derivation of proteins whose expression is increased in MPNST

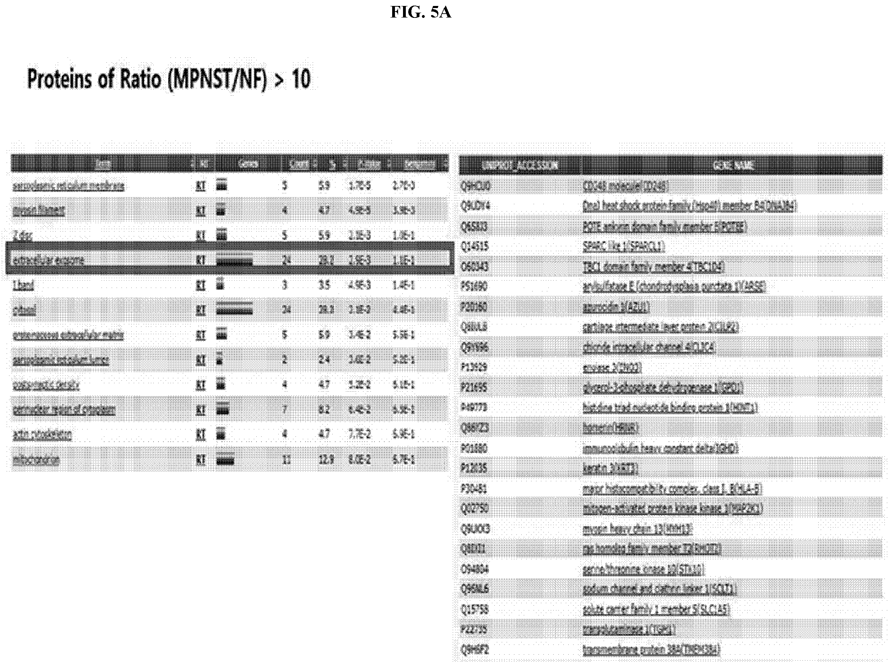

Through quantitative proteomic analysis using a volcano plot analysis, as shown in FIG. 5A, compared to benign NF, MPNST showed at least a 10-fold increase in expression, and proteins mainly expressed in extracellular exosomes were investigated (shown by a red box). This is because proteins excreted by exosomes can be detected in blood or urine, which is advantageous for collecting specimens for diagnosis.

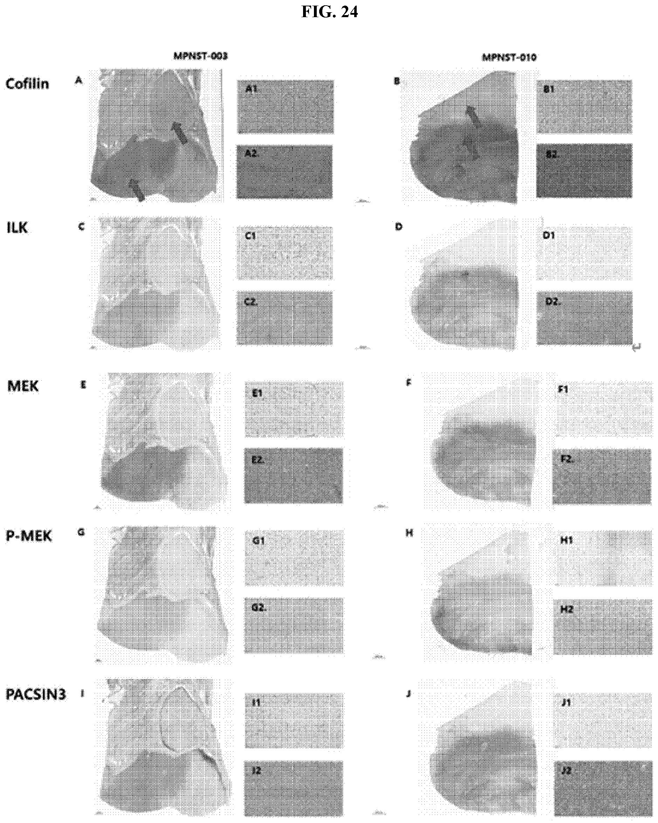

As a result, as shown on the right side of FIG. 5A, 24 types of proteins, for example, CD248, DNAJB4, POTEE, SPARCL1, and TBC1D4 were selected.

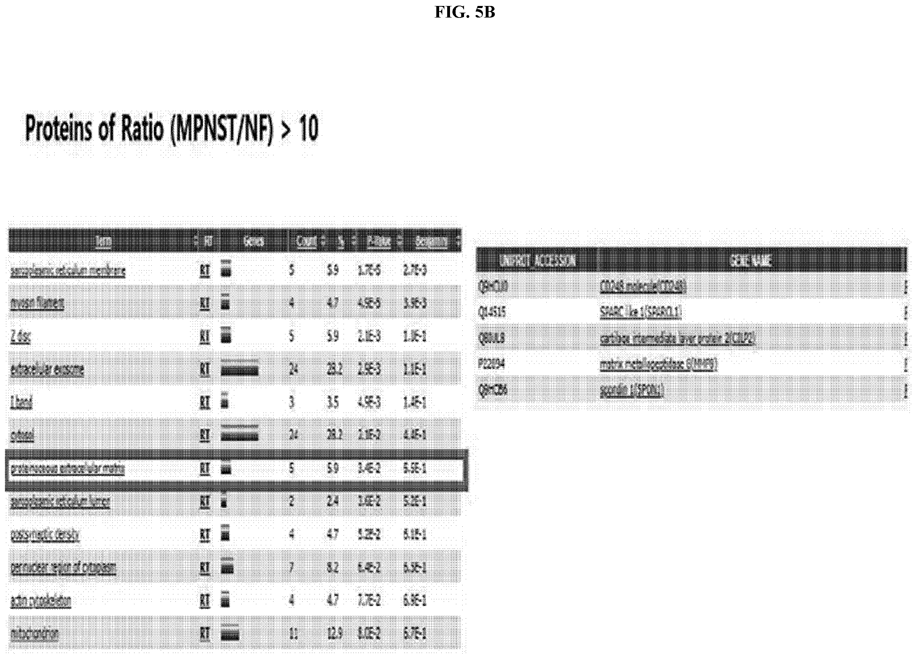

In addition, as shown in FIG. 5B, extracellular matrix-degrading proteins (proteinaceous extracellular matrix) were investigated (shown by a red box). This is because proteins expressed in the extracellular matrix can also be detected in blood or urine. In addition, matrix degradation is a phenomenon that occurs when cancer cells infiltrate surrounding tissue, so the proteins are based on cell malignancy.

As a result, as shown on the right side of FIG. 5B, CD248, SPARCL1, CILP2, MMP8, and SPON1 proteins were selected.

### Comparative Example 1. Investigation of expression pattern of S-100 protein in MPNST and benign NF

The expression pattern of S-100 protein, which has been conventionally used as an important pathological indicator for distinguishing benign NF and MPNSTs, was investigated.

As a result of investigating a total of nine S-100 protein isoforms, as shown in FIGS. 6A to 6I, there was a difference in expression pattern between benign NF and an MPNST according to individual patients. Such a result clearly reveals the limitations of the S-100 protein as a specific marker for distinguishing benign NF and MPNSTs.

In addition, as a result of investigating vimentin as another marker for distinguishing benign NF and MPNSTs, there was no difference in expression pattern between benign NF and MPNSTs.

Taken together, the above results show that the markers, S-100 and vimentin, which have been conventionally used, have clear limitations to being applied clinically as MPNST-specific biomarkers.

### Example 4. Selection of diagnostic marker for MPNST

### 4.1. Endosialin (CD248)

Endosialin, which is a protein encoded by the CD248 gene, is a molecule involved in vascular regeneration in tumors, and is secreted to the extracellular matrix and found in exosomes.

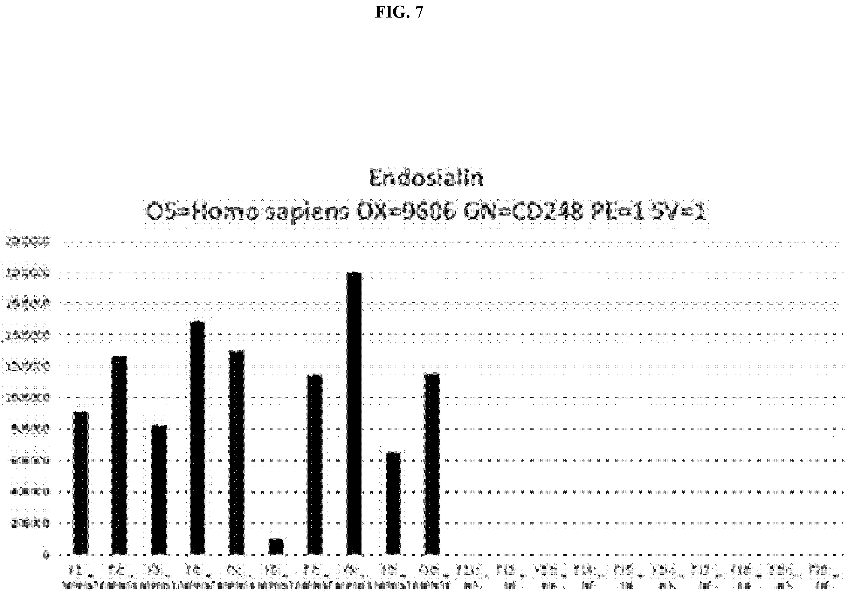

As confirmed in FIG. 7, endosialin was specifically found in all MPNST tissues obtained from five patients. Particularly, since endosialin is found in exosomes and thus is expected to be detected in blood and urine samples, it is expected to have high utility as an MPNST diagnostic marker.

### 4.2. SPARC-like protein 1 (SPARCL1)

SPARC-like protein 1 encoded by the SPARCL1 gene is a protein that is mainly secreted extracellularly, and binds to calcium, the extracellular matrix, and collagen.

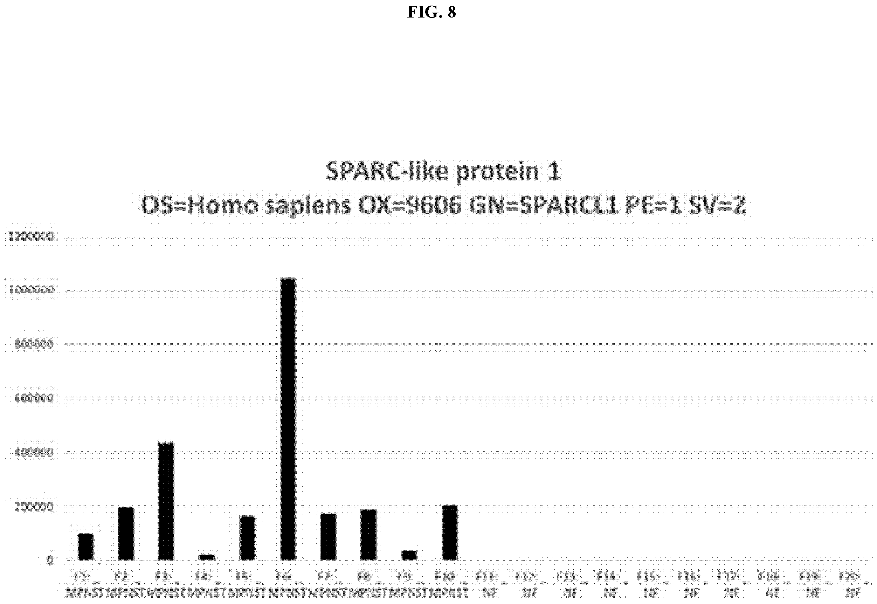

As shown in FIG. 8, it was confirmed that SPARC-like protein 1 is not detected in benign NF, but specifically expressed in MPNST. Since this protein is also found extracellularly and present in exosomes, it is expected to be detected in blood and urine samples, and thus is expected to have high utility as an MPNST diagnostic marker.

### 4.3. Neutrophil collagenase (MMP8)

Neutrophil collagenase encoded by the MMP8 gene is a protease degrading the extracellular matrix, and is known to be involved in cancer metastasis and various inflammatory responses.

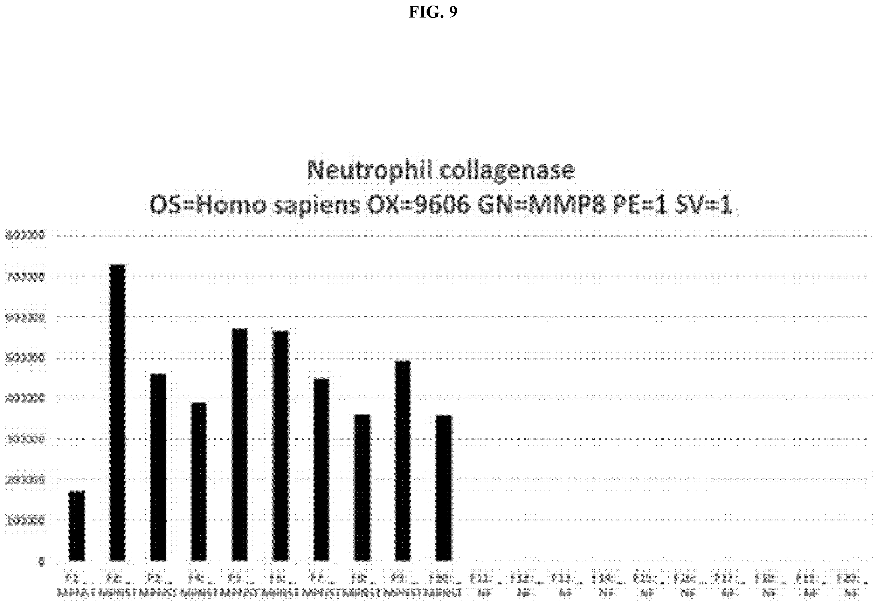

As shown in FIG. 9, it was confirmed that the neutrophil collagenase is specifically expressed in all MPNST tissues collected from five patients. Particularly, since neutrophil collagenase is secreted to the extracellular matrix, it is expected to be detected in blood and urine samples, and thus is expected to have high utility as an MPNST diagnostic marker.

### Example 5. Discovery of new MPNST therapeutic target

### 5.1. Hierarchical cluster analysis

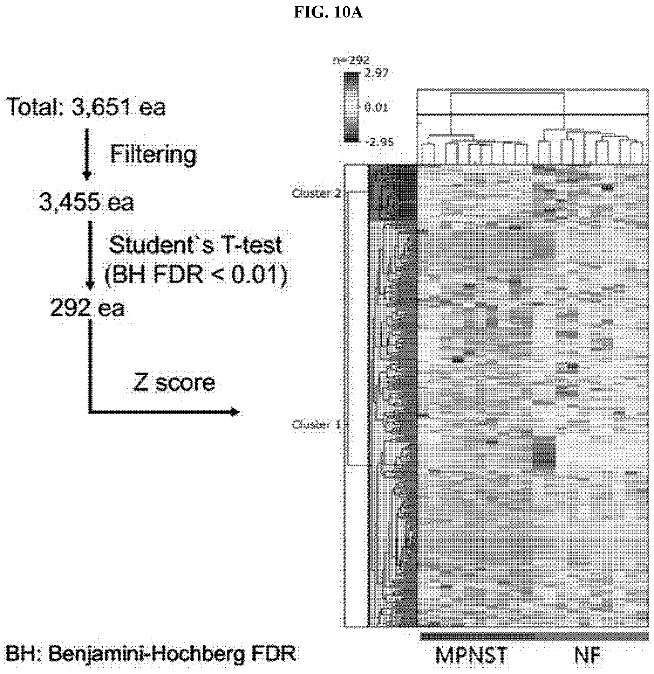

Proteomes whose expression is specifically increased in MPNSTs were classified into two clusters through hierarchical cluster analysis. More specifically, as shown in FIG. 10A, from a total of 3,624 proteins identified in the two groups, 3,455 proteins identified in 80% or more of the samples were extracted for each group, and the two groups were subjected to a Student's T test to select 292 proteins satisfying Benjamini-Hochberg FDR < 0.01. The Z scores for the quantitative information of the corresponding proteins were calculated and subjected to K-means clustering analysis, confirming that two clusters are formed. The proteins corresponding to each cluster were subjected to investigation of a biological process, a cellular compartment, and a molecular function using g:Profiler (https://biit.cs.ut.ee/gprofiler/).

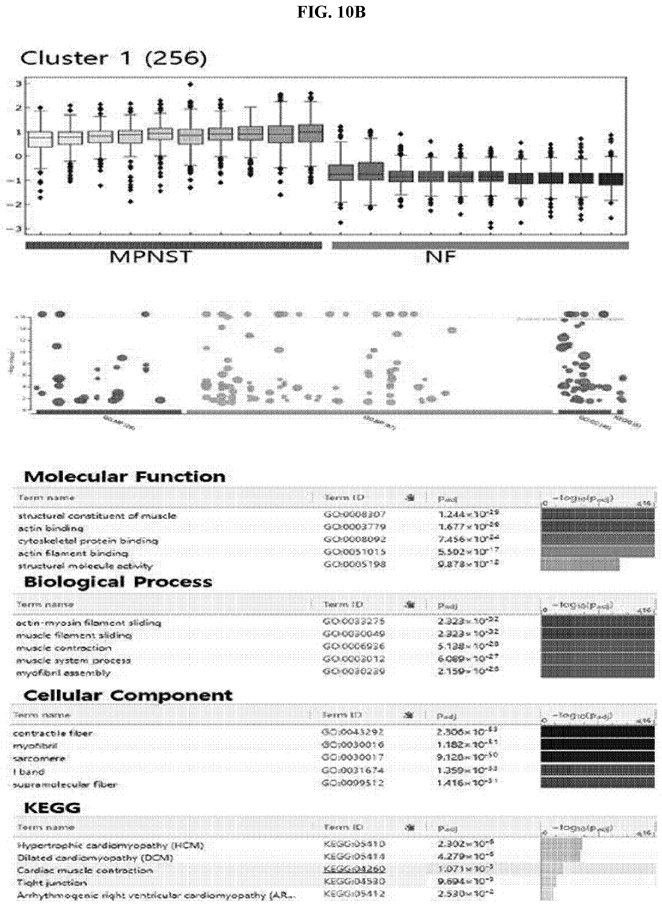

Referring to FIG. 10B, Cluster 1 includes proteins whose expression is increased in MPNSTs, in which proteins functioning by binding to an extracellular matrix, such as actin or musculoskeletal proteins were found in terms of function.

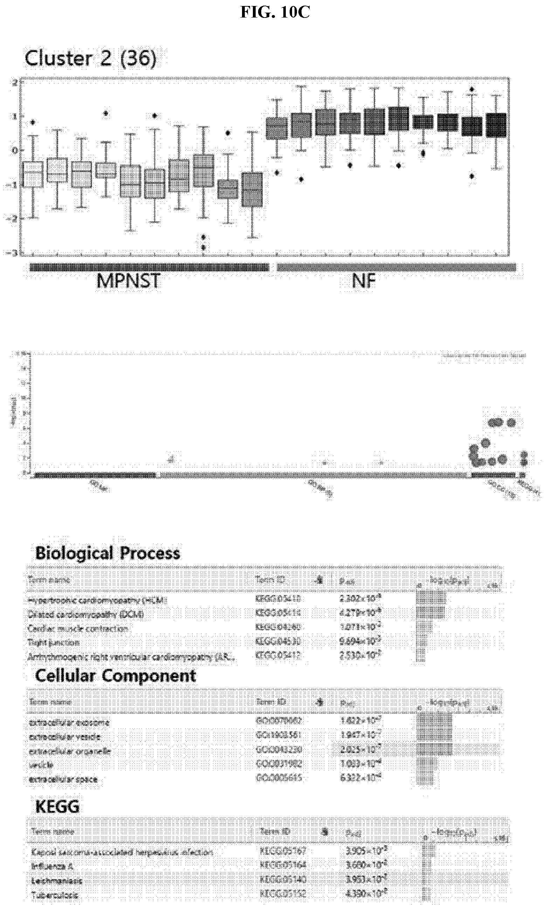

Referring to FIG. 10C, Cluster 2 includes proteins whose expression was decreased in MPNSTs, in which proteins usually secreted to the extracellular matrix or present in exosomes were found.

### 5.2. Discovery of target signaling pathway through GO analysis and ingenuity pathway analysis (IPA)

Gene ontology (GO) analysis and QIAGEN IPA were performed on 144 types of MPNST-specific proteins to confirm pathways involving proteins whose expression is specifically changed in MPNSTs. The accession numbers of the 144 types of MPNST-specific proteins were input to ShinyGO and IPA servers to investigate the biological processes, cellular compartments and molecular functions, which involve the 144 types of MPNST-specific proteins.

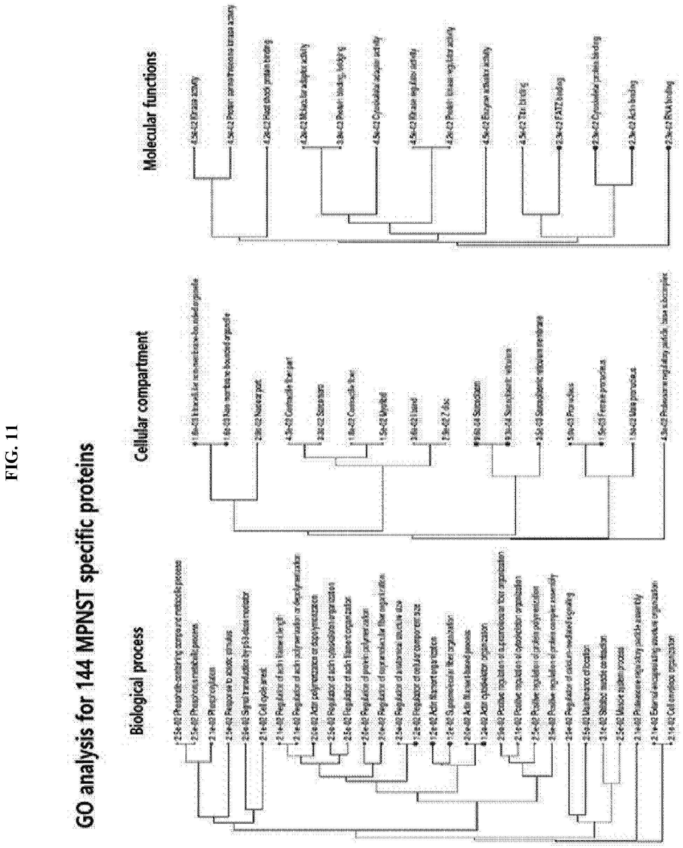

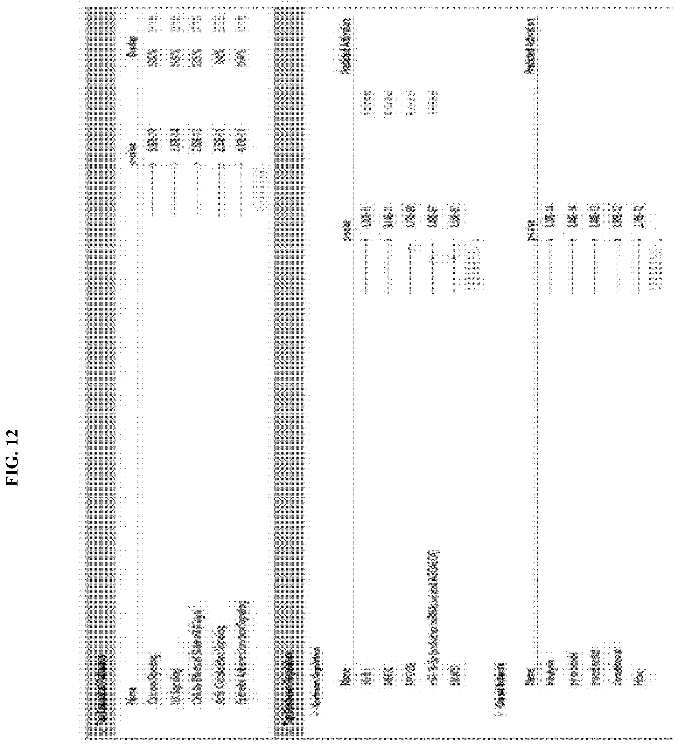

As a result, as shown in FIGS. 11 and 12, it was confirmed that the ILK signaling pathway, the actin-cytoskeleton signaling pathway, and the RhoGDI signaling pathway are activated specifically in MPNSTs. In addition, when inferring the upper-level signaling of the signaling pathway, it was predicted that this is based on the activation of TGF-β.

### 5.3. Discovery of kinase as new therapeutic target

In association with the signaling pathways discovered in Example 5.2, kinases whose expression or activity are specifically increased in MPNSTs can be used as therapeutic targets. Therefore, the present inventors discovered kinases whose expression levels are increased in MPNSTs.

As a result, as shown in FIGS. 13 to 21, it was confirmed that kinases such as CDK4, MAP2K1, PDK3, STK10, ALPK3, PHKG1, CDK5, PNKP, and PACSIN3 are increased in MPNSTs. Accordingly, inhibitors of these kinases, for example, antagonists against these kinases, may be used for MPNST treatment.

Particularly, since fostamatinib exhibits multiple antagonistic actions against CDK4, MAP2k1, STK1, and PHKG1, it is expected to be used as an MPNST therapeutic agent.

### Example 6. Verification of MPNST diagnostic marker

IHC staining was performed on tissue of MPNST patients to verify the expression of MMP8 protein among the MPNST diagnostic markers discovered in Example 4.

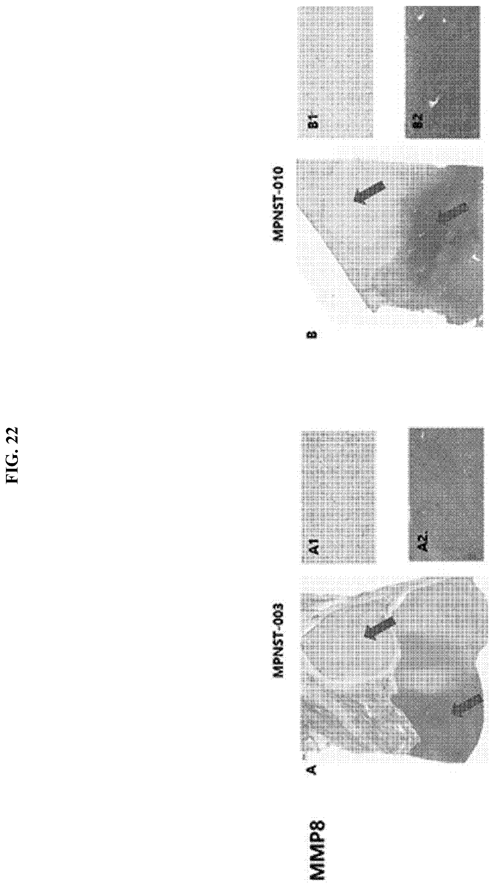

As a result, as shown in Table 4 and FIG. 22, compared to NF tissue, it was confirmed that the MMP8 protein (neutrophil collagenase) is overexpressed in MPNST tissue.

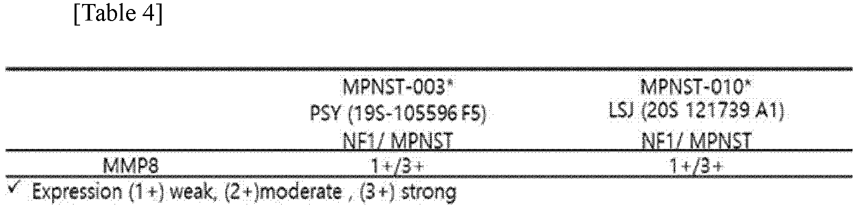

In addition, a difference in expression of MMP8 protein in blood, from which it is easy to obtain a patient's sample, was confirmed by ELISA. Specifically, ELISA was performed on plasmas of a normal group (WT00), an NF1 patient (SEL00), an MPNST patient with complete post-OP tumor removal among MPNST patients (clear), a patient who is likely to have tumors because the tumors are not completely removed depending on the pattern of tumor removal (intermediate), and a patient from which no tumors are removed or who has tumor metastasis (involvement).

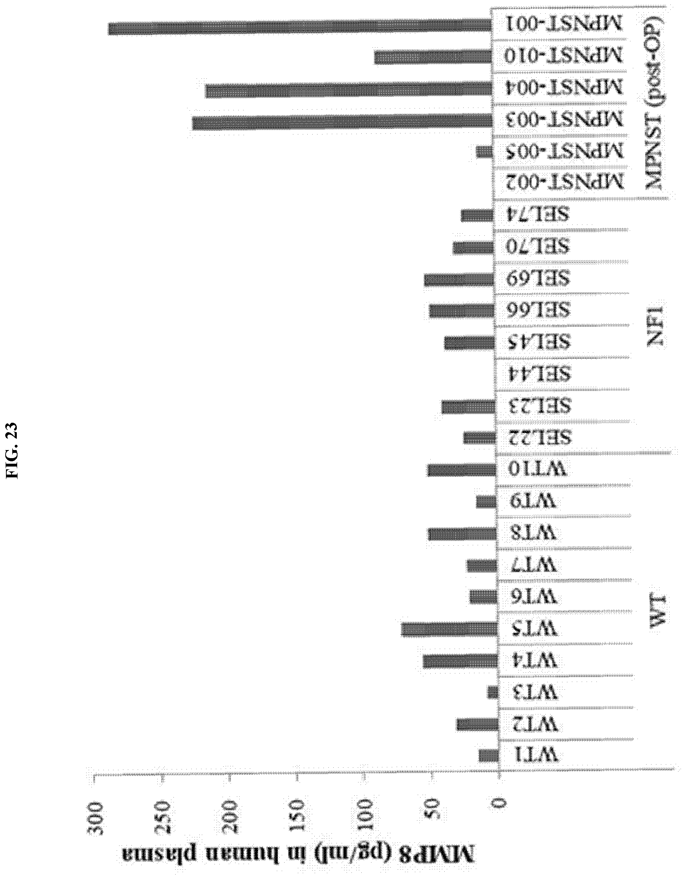

As a result, as shown in Table 5 and FIG. 23, it was confirmed that in the plasmas of the patient in which tumors are not completely removed (intermediate), and the patient in which metastasis occurs or no tumors are removed (involvement), MMP8 protein expression is increased. From the above results that confirm the difference in MMP8 protein expression according to the presence or absence of tumor removal, it can be confirmed that MMP8 can be used as an MPNST diagnostic marker.

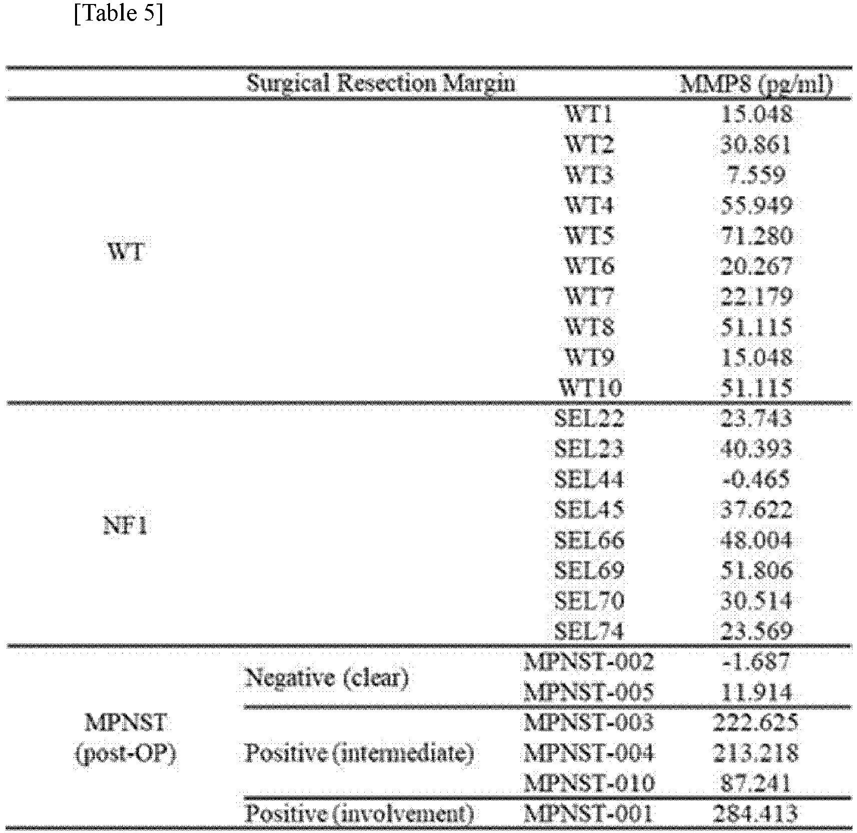

### Example. 7 Verification of MPNST therapeutic target

To present the abnormal activation degrees of the ILK signaling pathway, the actin-cytoskeleton signaling pathway, and various kinases, which are MPNST therapeutic targets, discovered in Example 5, IHC staining was performed on the ILK, Cofilin, MEK, p-MEK, and PACSIN3 proteomes in the tissue of MPNST patients.

As a result, as shown in Table 6 and FIG. 24, it was confirmed that the expression of the ILK, Cofilin, MEK, p-MEK, and PACSIN3 proteomes is increased in the MPNST tissue, compared to the NF tissue.

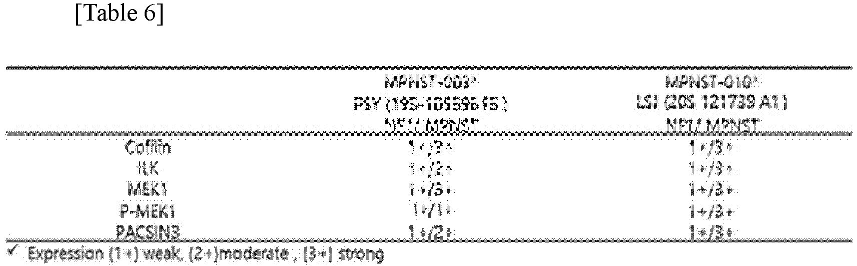

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

The present invention relates to a composition for differential diagnosis of malignant peripheral nerve sheath tumor (MPNST), and it is expected to be effectively used for rapidly prediction and diagnosis of whether a malignant tumor occurs in an NF-1 patient using the composition for detecting a protein marker of the present invention. Thus, the present invention has industrial availability.

## Claims

1. A composition for differential diagnosis of malignant peripheral nerve sheath tumor (MPNST), comprising:
an agent that measures the expression level(s) of one or more proteins selected from the group consisting of endosialin, SPARC-like protein 1, and neutrophil collagenase or the expression level of mRNA thereof as an active ingredient.

2. The composition of claim 1, which is able to diagnose and distinguish MPNST from neurofibromatosis type I (NF-1).

3. The composition of claim 1, wherein the agent for measuring the expression level of the protein is an antibody or aptamer specific for the protein.

4. The composition of claim 1, wherein the agent for measuring the expression level of the mRNA is a primer set or probe, which specifically binds to the mRNA.

5. A kit for differential diagnosis of malignant peripheral nerve sheath tumors (MPNSTs), comprising the composition of any one of claims 1 to 4.

6. The kit of claim 5, which comprises instructions for determining a case as MPNST when the expression of one or more proteins selected from the group consisting of endosialin, SPARC-like protein 1, and neutrophil collagenase or the expression of mRNA thereof is detected, or when the expression level thereof is higher than that of a benign neurofibromatosis sample.

7. A method of providing information required for the differential diagnosis of malignant peripheral nerve sheath tumor (MPNST), comprising:
measuring the expression level(s) of one or more proteins selected from the group consisting of endosialin, SPARC-like protein 1, and neutrophil collagenase, or the expression level of mRNA thereof in a biological sample isolated from a subject.

8. The method of claim 7, further comprising:
diagnosing a case as MPNST when the expression of one or more proteins selected from the group consisting of endosialin, SPARC-like protein 1, and neutrophil collagenase or the expression of mRNA thereof is detected, or when the expression level thereof is higher than that of a biological sample isolated from a neurofibromatosis type I (NF-1) patient.

9. The method of claim 7, wherein the subject is a patient suspected of having MPNST or diagnosed with NF-1.

10. The method of claim 7, wherein the biological sample is one or more selected from the group consisting of blood, whole blood, plasma, urine, tissue, cells, an organ, bone marrow, a microneedle aspiration specimen, a microneedle washing fluid, a core needle biopsy specimen, and saliva.

11. The method of claim 7, wherein the protein expression level is measured by one or more methods selected from the group consisting of western blotting, enzyme linked immunosorbent assay (ELISA), radioimmunoassay (RIA), radioimmunodiffusion, Ouchterlony immunodiffusion, Rocket immunoelectrophoresis, tissue immunostaining, immunoprecipitation assay, complement fixation assay, fluorescence-activated cell sorting (FACS) and a protein chip.

12. The method of claim 7, wherein the mRNA expression level is measured by one or more methods selected from the group consisting of PCR, RNase protection assay, northern blotting, southern blotting, in situ hybridization, and a DNA chip.

13. A method of screening a material for preventing, alleviating or treating malignant peripheral nerve sheath tumors (MPNSTs), comprising:
(a) bringing a candidate material into contact with isolated cells or tissue;
(b) measuring the activity of one or more signaling protein(s) involved in signaling pathway(s) selected from the group consisting of the TGF-β signaling pathway, the ILK signaling pathway, the actin-cytoskeleton signaling pathway, and the RhoGDI signaling pathway or an expression level of gene(s) encoding the protein(s) in the cells or tissue; and
(c) determining the candidate as a material for preventing, alleviating or treating MPNSTs when the activity of the protein or the expression level of a gene encoding the protein in cells or tissue in contact with the candidate material is lower than that before contact with the candidate material.

14. The method of claim 13, wherein the signaling protein is a kinase.

15. The method of claim 14, wherein the kinase is a kinase encoded by one or more genes selected from the group consisting of CDK4, STK10, CDK5, MAP2K1, ALPK3, PNKP, PDK3, PHKG1, CFL1, and PACSIN3.

16. The method of claim 13, wherein the isolated cells or tissue is nerve cells or nerve tissue.
